# EUROPEAN PATENT SPECIFICATION

(11) **EP 1 608 407 B1**
(45) Date of publication and mention of the grant of the patent: **30.08.2006**
(21) Application number: 04719030.1
(22) Date of filing: 10.03.2004
(51) Int. Cl.: A61K 47/44, A61K 45/06

(54) **DISPERSIBLE FORMULATION OF AN ANTI-INFLAMMATORY AGENT**
DISPERGIERBARE PHARMAZEUTISCHE ZUSAMMENSETZUNG EINES ENTZÜNDUNGSHEMMERS
FORMULATION DISPERSIBLE D'UN AGENT ANTI-INFLAMMATOIRE

(30) Priority: 20.03.2003 US 456325 P
(43) Date of publication of application: 28.12.2005
(73) Proprietor: Pharmacia Corporation, Chesterfield, MO 63017-1732 (US)
(72) Inventor: BRITTEN, Nancy, J. Pfizer Global Res, and Dev., Chesterfield, MO 63017-1732 (US); BURNS, John, W. Pfizer Global Res. and Dev., Chesterfield, MO 63017-1732 (US); HALLBERG, John, W. Pfizer Global Res. and Dev., Chesterfield, MO 63017-1732 (US); WALDRON, Niki, A. Pfizer Global Res. and Dev., Chesterfield, MO 63017-1732 (US); WATTS, Jeffrey, L. Pfizer Global Res. and Dev., Chesterfield, MO 63017-1732 (US)
(74) Representative: Rutt, Jason Edward
(86) International application number: PCT/IB2004/000826
(87) International publication number: WO 2004/082588

(56) References cited:
- WO-A-96/39146
- WO-A-98/25621
- GB-A- 1 370 699
- US-A1- 2001 049 366
- OWENS W E ET AL: "DETERMINATION OF MILK AND MAMMARY TISSUE CONCENTRATIONS OF CEFTIOFUR AFTER INTRAMAMMARY AND INTRAMUSCULAR THERAPY" JOURNAL OF DAIRY SCIENCE, vol. 73, no. 12, 1990, pages 3449-3456, XP002291861 ISSN: 0022-0302

## Description

### FIELD OF THE INVENTION

The present invention relates to a method of treatment and/or prevention of an inflammatory condition in a fluid-containing organ having a natural exterior orifice, such as the udder of a milk producing animal or an ear of a subject. The invention also relates to a dispersible pharmaceutical composition suitable for infusion into the organ according to the method of the invention, and a process for preparing such a composition.

### BACKGROUND OF THE INVENTION

Mastitis is an inflammation of the mammary gland of milk producing animals, for example dairy cows, most often caused by bacterial infection. Bacteria enter through the teat canal of the animal and can cause acute, clinical or sub-clinical mastitis. Over 135 organisms have been documented as causative pathogens for bovine mastitis. Three of the major groups of pathogens are gram-positive cocci, gram-negative bacilli and gram-positive bacilli. Hygiene, environmental factors and metabolic disturbances deriving from high milk yield combine to create conditions favorable to the onset of mastitis. An increased somatic cell count, associated with mastitis, is positively correlated with infection and negatively correlated with milk production. Frequently, an infected cow must be removed from the herd and dried up. Mastitis often affects a cow during its entire life unless the disease is properly treated. Infection rates average from 10% to 30% of the cows in a typical herd, with losses per cow ranging from $185 to $250 per cow per year. Bovine mastitis is the most economically costly disease to the dairy industry, with losses estimated at two billion dollars annually in the United States alone. The majority of these losses are due to reduced milk production.

Because inflammation and leukocytosis resulting from coliform mastitis often persist after the bacteria can no longer be isolated from the gland, the use of long term anti-inflammatory drug therapy can be useful in the treatment of mastitis.

Intramammary administration of dispersible compositions comprising an antibacterial agent for treatment of mastitis in milk producing animals is well known. Several compositions suitable for such administration are formulated as oil based formulations.

U.S. Patent No. 3,636,194 to Parizeau discloses a composition for treating mastitis by intramammary infusion, comprising an antibiotic, a vegetable oil, an alcohol-soluble fraction of natural lecithin phospholipid material for promoting dispersion of the oil in milk, the phospholipid being selected from the group consisting of phosphatidyl choline and phosphatidyl ethanolamine and mixtures thereof and present in an amount of at least 0.25% in said oil. Such compositions are said to provide rapid dispersion into milk and short milkout times.

British Patent Application No. 1,131,527 discloses a composition for treating mastitis comprising an active substance and a pharmaceutically acceptable oil base, said composition containing phospholipid material consisting substantially entirely of alcohol-soluble material for promoting dispersion of the composition in milk.

European Patent Application No. 0 222 712 discloses a composition which contains one or more antimicrobial agents dispersed in an oil consisting of a mixture of triglycerides of palmitic and stearic acid together with polyoxyethylenated cetyl alcohol and stearyl alcohol, and held in an oily medium of mineral, vegetable, synthetic or mixed extraction. Such compositions are said to speed up release of the antimicrobial agent in the udder, enhancing its biological potential, and reducing milkout time.

The use of anti-inflammatory agents to treat mastitis has also been proposed.

U.S. Patent No. 5,756,529 to Isakson & Talley discloses a method of using pyrazolyl benzenesulfonamide compounds to treat inflammation in a companion animal. Such compounds are said to be useful for treatment of pain, fever, joint disease, traumatic injury, arthritis, myositis, tendinitis, equine colic, mastitis, peritonitis, skin conditions, bums, gingivitis, hypersensitivity, conjunctivitis, eye inflammation, swelling and myocardial ischemia.

International Patent Publication No. WO 02/22107 discloses compositions comprising one or more bioactive agents in a liquid carrier, which has been modified to have an increased level of oxidation products, wherein the bioactive agents include anti-infectives, antineoplastics, immunomodulators, antipyretics, analgesics and anti-inflammatory agents (*e.g*., COX-2 inhibitors). Such compositions can be administered by a parenteral (*e.g*., subcutaneous, intramammary, intravenous, intraperitoneal or intramuscular), topical, intravaginal, oral or rectal route.

International Patent Publication No. WO 02/006865 discloses one or more bioactive substances in a non-aqueous carrier wherein the composition has been adjusted to have a water activity of between about 0.2 and about 0.5. Parenteral, topical, oral, intravaginal, rectal and intramammary routes of administration are proposed. Among the bioactive agents listed are anti-infectives, antineoplastics, immunomodulators, antipyretics, analgesics and anti-inflammatory agents (*e.g*., COX-2 inhibitors).

International Patent Publication No. WO 99/20259 discloses a combination of thiamphenicol and diclofenac for use in veterinary medicine to treat infections with associated inflammatory conditions.

International Patent Publication No. WO 01/60409 discloses a paste composition comprising a therapeutic agent, fumed silica, a viscosity modifier and a hydrophilic carrier; wherein the therapeutic agent is selected from insecticides, acaricides, parasiticides, antibiotics, growth enhancers, oil-soluble NSAIDs, avermectins, milbemycins, nordulisporic acid, estrogens, progestins, phenylpyrazoles, substituted pyridyl methyl derivatives and COX-2 inhibitors. Oral, topical, dermal and subdermal routes of administration are contemplated for the paste composition. Such compositions are said to have application in veterinary practice in treatment of diseases such as pneumonia, mastitis, metritis, rhinitis and bronchitis.

U.S. Patent Application Publication No. 2002/0032228 discloses use of a heterocycle containing compound, for example a diphenyl heterocycle derivative, to treat diarrheal diseases, whooping cough, anthrax, smooth muscle contraction conditions and mastitis. Celecoxib and rofecoxib are listed as preferred diphenyl heterocycle derivatives.

A Labrafil product brochure (Notice OL 0050/5th edition) from Gattefossé Corporation contains an extract from a thesis by Valette (1957), discussing characteristics of Labrafil^{™} M-1944CS in the ear canal. The same thesis describes an experiment involving injecting Labrafil^{™} M-1944CS mixed with gentian violet into a cow teat. It was shown that Labrafil^{™} wetted the entire surface of the mammary parenchyma section and reached the retromammary ganglion.

Two articles by Gao *et al.* (1995) in *Pharmaceutical Research* 12(6), 857-868, "Controlled release of a contraceptive steroid from biodegradable and injectable gel formulations: *in vitro* evaluation" and "Controlled release of a contraceptive steroid from biodegradable and injectable gel formulations: *in vivo* evaluation", describe preparation of gels containing levonorgestrel, Labrafil^{™} M-1944CS and glyceryl palmitostearate.

Otic disorders rank second only to the common cold as the most frequent illness among children in the United States. Most otic disorders are the result of a painful inflammatory response to infections, allergic reactions or trauma to the ear. An otic infection may be of bacterial, fungal or viral origin and determination of the precise etiology is not practical since the causative organism is often difficult to isolate and culture. Otitis externa (external ear infections), otitis media (middle ear infections) and otorrhea (otitis media with ruptured ear drum causing effusion) are among the most prevalent otic disorders.

Otitis externa, involving the ear canal portion of the external ear, is a common otological problem occurring mainly during hot, humid weather, and five times more frequently in swimmers than in non-swimmers. In the incipient stage, symptoms include itching and pain in the ear canal, and tenderness when pressure is applied around the external auditory canal, the ear lobe is pulled or the jaw is moved. In the definitive stage, suppuration occurs in the ear canal and hearing may be decreased. Over 90% of cases of otitis externa are due to bacterial and fungal infections.

Pathological conditions can arise from, and can cause, changes in the surface tension of air/liquid interfaces of tissue surfaces, especially epithelial surface tissues. The external auditory canal is lined with epithelium. The cerumen exudate, normally secreted upon the epithelial tissue lining the external auditory canal, imparts a particularly high surface tension thereto. Inflammatory by-products can further increase such surface tension. Increased surface tension is an important factor in both the symptoms and treatment of otitis. In addition, and even in the absence of canal closure, the increased surface tensions resident upon the epithelial lining of the outer ear canal, tends to inhibit uniform and/or effective application of therapeutic agents.

In the past, otitis externa has been treated with topical application of therapeutic agents demonstrating antimicrobial activity as well as anti-inflammatory action. Broad spectrum topically effective antibiotic otic suspensions containing antibacterial agents, for example neomycin sulfate, colistin sulfate, polymyxin B, or combinations thereof, all broad spectrum in effect, have been utilized to destroy causative bacteria. Antimycotic topically acting agents, for example nystatin and clotrimazole, have been employed to destroy underlying fungal disease. In addition, the antiviral agent acyclovir has been utilized to treat viral otitis externa including herpes zoster.

Anti-inflammatory agents including, for example, hydrocortisone, hydrocortisone acetate and dexamethasone sodium phosphate, often included in the topically acting suspensions identified above, have been employed to control the inflammatory process of otitis externa. Most often, antimicrobial and anti-inflammatory agents are utilized in combination to treat the causative, triggering disorder, *e.g*., bacterial infection, as well as the inflammatory process itself. They are also most often administered as suspensions in drop form for topical administration to the affected ear. In order to enhance and provide a more uniform delivery of such medications to the epithelial lining of the outer ear canal, wicks, made of absorbent material such as cotton, are utilized to draw the suspension into the ear canal. However, due to the exudate present in purulent forms of otitis externa, and the cerumen present in virtually all inflammatory conditions, high surface tension resists uniform distribution of such medications throughout the external auditory canal.

The most common otic disorder, otitis media, is a leading cause of hearing loss in the United States and represents a significant disability interfering with childhood learning processes. See Estrada (1997), *Infect. Med.* 14(3), 239-244. Otitis media accounts for over 35 percent of all childhood visits to pediatricians each year and represents more than $3.5 billion in U.S. health care costs annually.

During episodes of otitis media, the relatively high surface tensions present at the air/liquid interface located upon the epithelial lining of the tube lumen increase the opening pressure required to open this channel.

Typically otic infective disorders such as otitis media are treated with a course of antibiotic therapy. See *The Merck Manual,* 17th edition (1999), Section 7, Chapter 84. Systemic administration of antibiotics generally requires high initial doses and an appreciable lag time to achieve therapeutic levels in the ear. Systemic application of drugs via parenteral or oral routes, while eventually reaching the eustachian tube and middle ear, may have adverse systemic effects and, more importantly, are not especially effective at delivering a concentrated dose of the applicable drugs where they are truly needed, directly to the target tissues. At the same time, direct drug application has been complicated by the sealed chamber anatomy of the middle ear.

Combinations of antibacterial and anti-inflammatory agents, formulated together in a pharmaceutically acceptable vehicle, have been proposed for topical application to the ear, in various patents and publications including those individually cited below.

U.S. Patent No. 6,395,746 to Cagle *et al.*

U.S. Patent No. 6,440,964 to Cagle *et al.*

U.S. Patent No. 6,509,327 to Cagle *et al.*

U.S. Patent No. 5,679,665 to Bergamini *et al.*

U.S. Patent No. 5,965,549 to Purwar & Goldman.

U.S. Patent Application Publication No. 2001/0049366.

U.S. Patent Application Publication No. 2002/0142999.

U.S. Patent Application Publication No. 2002/0044920 discloses treating immune-mediated ear disorders by administering a TNF antagonist and a pyrimidine synthesis inhibitor with a steroid, an anti-inflammatory compound (for example an NSAID or a COX-2 inhibitor), a cytotoxic compound, an anti-neoplastic metabolite, or a secondary antirheumatic agent.

U.S. Patent Application Publication No. 2002/0076383 discloses administration of a composition as an aerosol through the external auditory canal, the composition comprising a lipid surfactant in an amount effective in lowering surface tension of an air/liquid interface upon epithelial tissue lining, a spreading agent and a propellant, wherein the spreading agent is selected from the group consisting of lipids, sterols, fatty acid, cholesterol esters, phospholipids, carbohydrates and proteins, all in powder form. The composition is said to increase external auditory canal patency while providing protection against occurrence of otitis externa.

U.S. Patent Application Publication No. 2002/0064503 discloses administration of a composition as an aerosol through an external airway, wherein the composition comprises a lipid surfactant in an amount effective in lowering surface tension of an air/liquid interface upon epithelial tissue lining, and a spreading agent selected from a group consisting of sterols, lipids, fatty acids, cholesterol esters, phospholipids, carbohydrates and proteins, all in powder form. The composition is said to increase the patency and pressure equalization performance of the eustachian tube lumen.

Ear drops have been contemplated as a formulation type for selective COX-2 inhibitors, for example in the patents and publications individually cited below.

U.S. Patent Application No. 2001/0041726.

U.S. Patent Application No. 2001/0053764.

U.S. Patent Application No. 2002/0010146.

U.S. Patent Application No. 2002/0013318.

U.S. Patent No. 6,307,047 to Black *et al.*

U.S. Patent No. 6,329,526 to Adams *et al.*

Despite recent advances that have been made in understanding the causes of otic disorders, they remain largely unpreventable and are difficult to effectively treat. It would be useful, therefore, to provide efficacious methods and compositions for the prevention and treatment of otic disorders and complications related thereto.

The most commonly used packaging containers and delivery devices for compositions intended for intramammary administration to treat or prevent mastitis in milk producing animals as well as for compositions for otic administration to treat otic disorders are constructed of oxygen-permeable plastic materials, for example polyethylene, polypropylene, etc. and mixtures thereof. The use of oxygen-permeable packaging containers and delivery devices for anti-mastitis compositions and for compositions for treatment or prevention of otic disorders poses serious problems for long term chemical and/or physical stability of a composition contained therein, if the composition comprises an ingredient, for example an active medicament or an excipient, that is prone to oxidative degradation.

Although the references cited above disclose a number of compositions for treatment of mastitis or for treatment of otic disorders, none addresses the problem of providing extended chemical and/or physical stability of a composition packaged in an oxygen-permeable container, where the composition comprises a pharmaceutically active agent and/or excipient that is prone to oxidative degradation. Despite the above teachings, there still exists a need in the art for pharmaceutical compositions having one or more of the following advantages over prior art compositions used in treatment of mastitis or over prior art compositions used in treatment or prevention of otic disorders: (a) extended chemical and/or physical stability even when packaged in oxygen-permeable containers and delivery devices, particularly where the composition comprises a pharmaceutically active agent or excipient that is prone to oxidative degradation, (b) safe, effective treatment of the inflammatory component of mastitis or of an otic disorder, (c) safe, effective treatment of the pain, inflammation, fever and swelling associated with mastitis or an otic disorder, (d) minimal to no irritation after administration of the composition, (e) rapid dispersibility of an anti-mastitis composition in milk and in udder fluids to quickly achieve efficacious medicament levels at sites of inflammation, (f) rapid dispersibility of an otic composition in the waxy moist environment of an ear to quickly achieve efficacious medicament levels at sites of inflammation, (g) a lowering of the surface tension of the air/liquid interface of epithelial tissue, increasing patency of the auditory canal, and (h) a protective coating for inflamed mucous membranes of the ear.

### SUMMARY OF THE INVENTION

Novel methods of treatment and pharmaceutical compositions having some or all of the advantageous attributes described above have now been developed. In particular, there is provided a novel method of treatment and/or prevention of an inflammatory condition in a fluid-containing organ having a natural exterior orifice, for example an udder of a milk-producing animal or an ear of a human or animal subject. The method comprises administering an anti-inflammatory agent to the organ via the exterior orifice. The anti-inflammatory agent is administered as a pharmaceutical composition comprising, in addition to said agent, a vehicle that comprises (a) an amphipathic oil that is water dispersible and ethanol insoluble, (b) microcrystalline wax and (c) a pharmaceutically acceptable non-aqueous carrier.

Such a composition has low interfacial tension when placed in contact with an aqueous medium. It is believed, without being bound by theory, that this low interfacial tension results in the composition dispersing readily in udder fluids such as milk as well as in the more waxy moist environment of an ear. In a preferred method of the invention, therefore, upon administration to the fluid-containing organ, the composition disperses in the fluid.

A preferred anti-inflammatory agent for use in the method of the invention is a selective COX-2 inhibitor.

The method can, for example, comprise intramammary infusion of such a composition for treatment of mastitis or other diseases of the udder in a milk producing animal, or otic infusion of such a composition for treatment and/or prevention of otic disorders, and is efficacious in a wide variety of inflammatory disorders which can be associated with a wide variety of infectious organisms. The term "infusion" herein embraces any operation wherein a liquid composition is caused to flow into the fluid-containing organ via the exterior orifice, for example the teat canal in the case of intramammary infusion or the external auditory canal in the case of otic infusion, regardless of the timescale involved. In the present context, "infusion" and "injection" are substantially synonymous. For example, the composition can be intramammmarily administered by inserting the cannula nozzle of a mastitis syringe into the external orifice of a teat canal and injecting the composition through the nozzle into the udder.

In another embodiment the anti-inflammatory agent, for example a selective COX-2 inhibitor, can be administered in combination therapy with a second agent. The second agent can be any therapeutically active agent useful in treatment of mastitis or otic disorders. Such second agents include, without limitation, antibacterial agents, antineoplastic agents, antipyretics, analgesics and the like, and combinations thereof.

The second agent can be administered by a route that is other than the route of administration of the anti-inflammatory agent. Alternatively, both agents can be administered by the same route, *i.e.*, via the exterior orifice of the organ, for example the teat canal in the case of an udder or the external auditory canal in the case of an ear. Where administration is by the same route, it is preferred that both agents be administered by intramammary or otic infusion in the form of a liquid composition comprising a vehicle as described above. It is especially preferred that the anti-inflammatory agent and the second agent be administered in a single composition containing both agents.

Accordingly, there is further provided a pharmaceutical composition comprising a vehicle that comprises (a) an amphipathic oil that is water dispersible and ethanol insoluble, (b) microcrystalline wax and (c) a pharmaceutically acceptable non-aqueous carrier. The vehicle has stably dispersed therein an anti-inflammatory agent, for example a selective COX-2 inhibitor, in an anti-inflammatorily effective amount.

In one embodiment the anti-inflammatory agent and/or an excipient in the composition is prone to oxidative degradation, and the composition exhibits extended chemical and/or physical stability when packaged in a container or delivery device having an oxygen permeable wall.

The novel composition has a low interfacial tension in aqueous fluids, thereby increasing dispersibility of the composition in milk and udder fluids, as compared to a conventional oil based formulation. This results in rapid distribution of the composition throughout the udder and thereby allows the anti-inflammatory agent and/or the second agent to reach inflamed tissue quickly, providing an efficacious level of medicament at a site of inflammation. The interfacial tension of a composition in an aqueous fluid determines the energy needed for dispersion and spreading of the composition in the fluid, as well as the energy necessary for a suspended particle in the composition to cross the oil/milk or oil/udder fluid interfacial boundary.

The low interfacial tension of the composition also increases dispersibility of the composition in the waxy moist environment of an ear, as compared to a conventional composition. The resulting rapid distribution of the composition throughout mucous membranes and lipid containing wax of the ear canal allows the anti-inflammatory agent and/or the second agent to reach inflamed tissue quickly, providing an efficacious level of the medicament at the site of inflammation. Such a composition can also produce a protective coating for inflamed mucous membranes of the ear.

Preferably the method or composition provides effective treatment of the inflammatory component of a mammary or otic disorder. Preferably the method or composition provides effective treatment and/or prevention of pain, inflammation, swelling and/or fever associated with such a disorder.

When administered by intramammary infusion, for example in treatment of mastitis, preferred methods and compositions can have additional advantages. For example, a preferred method enables suitably short milkout times. Milkout time for a lactating cow is the period of time from administration of a mastitis treatment to resumption of production of saleable milk. Following such administration, the concentration of active agent(s) in milk must fall to a level acceptable to the appropriate regulatory body before the milk is deemed suitable for human consumption. A suitably short milkout time reduces monetary losses to a dairy farmer caused by a mastitis outbreak.

Alternatively or in addition, a preferred method enables a low milk withholding time post calving after dry cow mastitis treatment, with no active agent residues in the offspring.

Alternatively or in addition, a preferred method enables a zero day slaughter meat withdrawal period following mastitis treatment. This attribute is especially important since it allows a farmer to dispose of a treated cow at any time it is financially advantageous to do so, rather than being required to keep and feed a cow for a specified amount of time after its treatment.

When administered by otic infusion, for example in treatment of infective disorders of the ear having an inflammatory component, preferred methods and compositions can have additional advantages. For example, a preferred method increases patency of the auditory canal and thereby reduces resistance to conduction of sound, improving the clarity and sensitivity of hearing.

Alternatively or in addition, a preferred method provides a coating on the epithelial lining of the ear that protects against deleterious effects of water and waterborne toxins, irritants and antigenic materials, and helps prevent otic disorders.

A further benefit of methods and compositions of the invention, whether for intramammary or otic use, is that they permit targeted delivery of at least the anti-inflammatory agent to the site of inflammation. Where a composition of the invention is used comprising both an anti-inflammatory agent and a second agent as defined herein, targeted delivery of both agents is provided to the site of infection and/or inflammation.

A still further benefit of preferred compositions, whether for intramammary or otic administration, is that they cause minimal to no irritation after administration.

A still further benefit of a composition of the invention is improved physical stability when compared to conventional oil and aqueous compositions, for example by virtue of improved composition resuspendability. A composition of the invention has been shown to cause flocculation of certain drugs, thereby improving resuspendability and eliminating the problem of suspension caking and possible delivery of a subpotent or non-efficacious dose.

A process is provided for preparing a pharmaceutical composition of the invention. The process comprises mixing, in any suitable order, an amphipathic oil that is water dispersible and ethanol insoluble, microcrystalline wax, a pharmaceutically acceptable non-aqueous carrier and an anti-inflammatory agent, for example a selective COX-2 inhibitor, to provide the composition, such a composition preferably having extended chemical and/or physical stability as described herein.

The present invention thus provides solutions to several long standing problems in the art and possesses one or more advantages over methods and compositions of prior art. Other features, advantages and benefits of the invention will be apparent from the description that follows.

### DETAILED DESCRIPTION OF THE INVENTION

The invention provides a method of treatment and/or prevention of an inflammatory condition in a fluid-containing organ having a natural exterior orifice, the method comprising administering an anti-inflammatory agent to the organ via the exterior orifice, in the form of a pharmaceutical composition comprising the anti-inflammatory agent in an anti-inflammatorily effective amount and a vehicle that comprises (a) an amphipathic oil that is water dispersible and ethanol insoluble, (b) microcrystalline wax and (c) a pharmaceutically acceptable non-aqueous carrier.

The method is particularly useful in treatment and/or prevention of inflammation accompanying an infective condition. An "infective condition" herein includes any disease, disorder or condition mediated by a pathogenic bacterium or that is otherwise responsive to treatment with an antibacterial agent such as an antibiotic drug.

It will be understood that reference herein to methods involving and compositions comprising "an anti-inflammatory agent" embraces such methods and compositions wherein more than one anti-inflammatory agent is used. Further, more than one therapeutically active agent other than an anti-inflammatory agent can optionally form the "second agent" herein.

The term "anti-inflammatorily effective amount" as used herein refers to an amount of an anti-inflammatory agent that is sufficient, when administered by the method of the invention, to reduce, relieve, prevent or delay onset of one or more symptoms of an inflammatory condition being treated.

A fluid-containing organ as contemplated herein includes a mammary organ, for example an udder of a milk producing animal such as a cow, a goat or a sheep. A "milk producing animal" can be a female of any mammalian species but is preferably an animal raised for the purpose of providing milk, *e.g*., a cow, a goat or a sheep, and encompasses such animals whether or not they are lactating at the time of the inflammatory and/or infective condition or at the time of treatment. The natural exterior orifice of the mammary organ is the orifice of the teat canal. A fluid-containing organ also includes an ear of a human or animal subject. The natural exterior orifice of the ear is the orifice of the external auditory canal.

The invention further provides a method of treatment of an inflammatory condition in a fluid-containing organ having a natural exterior orifice, the method comprising administering an anti-inflammatory agent to the organ via the exterior orifice and administering in combination therapy therewith a second agent as defined herein in therapeutically effective amounts of each; wherein the anti-inflammatory agent is administered as a pharmaceutical composition comprising said agent and a vehicle that comprises (a) an amphipathic oil that is water dispersible and ethanol insoluble, (b) microcrystalline wax and (c) a pharmaceutically acceptable non-aqueous carrier.

The term "combination therapy" herein means a treatment regimen wherein the anti-inflammatory agent and the second agent are administered individually or together in such a way as to provide a beneficial effect from co-action of these therapeutic agents. Such beneficial effect can include, but is not limited to, pharmacokinetic or pharmacodynamic co-action of the therapeutic agents. Combination therapy can, for example, enable administration of a lower dose of one or both agents than would normally be administered during monotherapy, thus decreasing risk or incidence of adverse effects associated with higher doses. Alternatively, combination therapy can result in increased therapeutic effect at the normal dose of each agent in monotherapy. "Combination therapy" herein is not intended to encompass administration of two or more therapeutic agents as part of separate monotherapy regimens that incidentally and arbitrarily result in sequential or simultaneous treatment.

Administration of the anti-inflammatory agent and the second agent typically is carried out over a defined time period (usually minutes, hours, days or weeks depending upon the combination selected). These therapeutic agents can be administered in a sequential manner, that is, at different times, typically separated by no more than about 24 hours, or in a substantially simultaneous manner.

When administered simultaneously, the anti-inflammatory agent and the second agent can be administered in separate dosage forms or in coformulation, *i.e.,* in a single dosage form. When the two agents are administered sequentially or in separate dosage forms, the second agent can be administered by any suitable route and in any pharmaceutically acceptable dosage form, for example by a route and/or in a dosage form other than that used for the anti-inflammatory agent. Alternatively, the second agent, like the anti-inflammatory agent, can be dispersed in a vehicle that comprises (a) an amphipathic oil that is water dispersible and ethanol insoluble, (b) microcrystalline wax and (c) a pharmaceutically acceptable non-aqueous carrier and administered via the natural exterior orifice of the fluid-containing organ. In a preferred embodiment, both agents are co-dispersed in the same vehicle and administered in a single operation.

Preferably the second agent is formulated in a pharmaceutically acceptable vehicle, and both the anti-inflammatory agent and the second agent are administered into the same fluid containing organ, for example by intramammary or otic infusion. A pharmaceutically acceptable carrier or vehicle is one that has no unacceptably injurious or toxic effect on the subject when administered as a component of a composition in an amount required herein. No excipient ingredient of such a carrier or vehicle reacts in a deleterious manner with another excipient or with the therapeutic agent(s) in a composition.

The pharmaceutical composition comprising the anti-inflammatory agent is a liquid injectable or infusable composition having said agent dispersed in a vehicle as described herein. The term "dispersed" herein means dissolved (*i.e.,* molecularly dispersed) or colloidally dispersed, for example as an emulsion or suspension. Typically the anti-inflammatory agent is suspended in solid particulate form in the vehicle.

The vehicle comprises three essential ingredients, optionally together with additional ingredients.

The first of these essential ingredients is an amphipathic oil that is water dispersible and ethanol insoluble. An "amphipathic oil" is defined as a substance having a molecular structure with a distinctly polar region and a distinctly non-polar region. Structurally these two regions of the amphipathic oil are sufficiently far apart that the unique properties of the two regions are distinctly separate. The term "ethanol insoluble" means that the amphipathic oil is essentially insoluble in ethanol at 20°C.

The second essential ingredient of the vehicle is microcrystalline wax.

The third essential ingredient of the vehicle is a pharmaceutically acceptable non-aqueous carrier. Such a carrier is typically an oil, as described more fully hereinbelow.

The selection of vehicle components is important in providing a composition that, upon administration to the fluid-containing organ, disperses in the fluid. It is believed, without being bound by theory, that such dispersion in the fluid within the organ results in targeted delivery of the anti-inflammatory agent and, optionally, the second agent, to the site of inflammation in the organ.

Where the method of the invention comprises injection or infusion of the composition into an udder via the teat canal, it can provide effective treatment of mastitis, other diseases of the udder, and/or a condition associated with a mammary disease. "Intramammary infusion" is an operation wherein a liquid composition is caused to flow into an udder via a teat canal, regardless of the timescale involved. In the present context, "infusion" and "injection" are substantially synonymous.

Where the method of the invention comprises injection or infusion of the composition into an ear via the external auditory canal, a process described herein as "otic infusion" regardless of the timescale involved, it can provide effective treatment and/or prevention of an otic disorder and/or a complication associated therewith. The subject suffering such otic disorder or complication associated therewith can be a human, companion animal, horse, farm livestock or the like.

Examples of such otic disorders include, but are not limited to, otitis externa (external ear infections), otitis media (middle ear infections), including acute, secretory, serous and chronic forms of otitis media, otorrhea (otitis media with ruptured ear drum causing effusion), acute mastoiditis, infections related to otic surgical procedures (such as tympanostomy and the like), otosclerosis, otalgia, otic pain, otic inflammation, otic bleeding, Lermoyez's syndrome, Meniere's disease, vestibular neuronitis, benign paroxysmal positional vertigo, herpes zoster oticus, Ramsay Hunt's syndrome, viral neuronitis, ganglionitis, geniculate herpes, labyrinthitis, including purulent labyrinthitis and viral endolymphatic labyrinthitis, perilymph fistulas, presbycusis, drug-induced ototoxicity, acoustic neuromas, aerotitis media, infectious myringitis, bullous myringitis, otic neoplasm, squamous cell carcinoma, basal cell carcinoma, other otic cancers, pre-cancerous otic conditions, nonchromaffin paragangliomas, chemodectomas, glomus jugulare tumors, glomus tympanicum tumors, perichondritis, aural eczematoid dermatitis, malignant external otitis, subperichondrial hematoma, ceruminomas, impacted cerumen, sebaceous cysts, osteomas, keloids, tinnitus, vertigo, tympanic membrane infection, tympanitis, otic furuncles, petrositis, conductive and sensorineural hearing loss, epidural abscess, lateral sinus thrombosis, subdural empyema, otitic hydrocephalus, Dandy's syndrome, bullous myringitis, diffuse external otitis, foreign bodies, keratosis obturans, otomycosis, trauma, acute barotitis media, acute eustachian tube obstruction, a complication associated with any of the above infections (such as hearing loss, brain abscess, fever, cholesteatomas, calcification of the middle and inner ear, ruptured ear drum, meningitis, facial paralysis and the like), postsurgical otalgia and the like.

The method of the invention is particularly suitable for treatment of otitis externa, otitis media, otorrhea, and infections having an inflammatory component that are related to an otic surgical procedure.

In one embodiment the otic disorder is a neoplasia. Examples of such neoplasia include, but are not limited to, otic neoplasia, squamous cell carcinoma, basal cell carcinoma, malignant external otitis, malignant nonchromaffin paraganglioma, malignant jugulare tumor, malignant glomus tympanicum tumor, a pre-cancerous otic condition and the like.

Combination therapy of the anti-inflammatory agent together with the second agent provides enhanced treatment options as compared to administration of either agent alone. As indicated above, the anti-inflammatory agent is dispersed in a vehicle that comprises (a) an amphipathic oil that is water dispersible and ethanol insoluble, (b) microcrystalline wax and (c) a pharmaceutically acceptable non-aqueous carrier, and is administered for example by intramammary or otic infusion, while the second agent is formulated into any acceptable immediate release or sustained release pharmaceutical dosage form. Suitable dosage forms for the second agent include, but are not limited to, a suspension, solution, emulsion, tablet, capsule, pill, powder, granules, elixir, tincture, syrup, lozenge, dragee, gel, ointment, spreadable paste, slurry, aerosol spray, ear drops, nasal drops, eye drops, suppository, implant and the like, and can be administered via any route including, but not limited to, oral, including peroral and intraoral, *e.g*., sublingual, buccal, etc.; parenteral, *e.g*., intramuscular, subcutaneous, intravenous, intraperitoneal, intra-articular, intradermal, intraspinal, intrasternal, intramedullary, intrasynovial, intrathecal, intracardiac, intraventricular, intracapsular, intracranial, etc.; intramammary, topical, transdermal, intranasal, otic, mucosal, rectal, intravaginal, pulmonary and the like.

Optionally, administration of the therapeutic agents described above can take place in further combination with other biologically active agents and non-drug therapies.

In all embodiments of the invention, at least the anti-inflammatory agent is administered locally. An advantage of such local administration is that the anti-inflammatory agent is preferentially directed toward its site of action, resulting in more rapid onset of therapeutic action and more complete delivery to the site of inflammation, compared with other routes of administration such as intramuscular, subcutaneous and oral routes. Local administration can allow the total therapeutic dose for a given effect to be decreased and avoids the hepatic first pass effect. In addition, local administration decreases or eliminates secondary effects, especially those linked to the active agent, at sites other than the site of infection. Local administration of an active agent can also improve its therapeutic index by decreasing its general toxicity and minimizing risk of undesirable systemic effects. Therapeutic index is a measure of the margin between a therapeutically effective dose and a toxic dose of a drug and is typically expressed as the ratio of LD₅₀ (a dose lethal to 50% of a population) to ED₅₀ (a dose therapeutically effective in 50% of the population).

The invention provides, in a further embodiment, a pharmaceutical composition adapted for intramammary and/or otic infusion, comprising a vehicle that comprises (a) an amphipathic oil that is water dispersible and ethanol insoluble, (b) microcrystalline wax and (c) a pharmaceutically acceptable non-aqueous carrier; the vehicle having stably dispersed therein an anti-inflammatory agent, for example a selective COX-2 inhibitor, in an anti-inflammatorily effective amount.

Preferably such a composition lowers the high surface tension of the air/liquid interface of epithelial tissues associated with an otic disorder, so as to increase patency of the auditory canal. A decrease in the surface tension of the air/liquid interface of the epithelium lining can minimize fluid accumulation, and in some instances enable evacuation of fluids held in the canal due to elevated surface tensions therein, and/or allow separation of the proximal and opposing epithelial walls of the auditory canal (often brought closer together due to elevated surface tension of the tissues) thereby improving conduction of sound. The term "increase patency" as used herein refers to opening, and reduction or elimination of blockage, of the auditory canal so as to form a patent conduit. Resistance to conduction of sound results from reduction of the volume, partial obstruction, or complete occlusion of the auditory canal due to swelling of the epithelial walls as a result of inflammation, the accumulation of increased amounts of cerumen secreted thereupon, and/or collection of fluids therewithin, including fluids containing waste products of the immune response or exogenous water.

In a particular embodiment of the invention an ingredient of the composition (the anti-inflammatory agent and/or a second agent and/or an excipient ingredient) is prone to oxidative degradation. Such a composition exhibits extended chemical and/or physical stability even when packaged in an oxygen permeable container or delivery device. The term "extended chemical and/or physical stability" herein means that a composition of the present embodiment has greater chemical and/or physical stability than a reference composition comprising the same medicament at the same concentration. A "reference composition" in the present context means a composition lacking one or both of the amphipathic oil and the microcrystalline wax, but otherwise similar to the composition of the invention.

Oxygen permeable containers or delivery devices can be made of any suitable thermoplastic material. Examples of such materials include, but are not limited to, polymers and copolymers of polystyrene, polyacrylonitrile, polyvinyl chloride, and particularly polyolefins. Polyolefins include, for example, polyethylene, polypropylene, polybutenes, polyisoprenes, polypentenes, copolymers thereof and mixtures thereof.

Compositions for intramammary administration are commonly packaged in syringes that are provided with a cannula nozzle for insertion into the teat to allow extrusion of the composition directly into the mammary gland via the teat canal. Intramammary suspension formulations are generally prepared in thickened vehicles to prevent settling of drug particles in the cannula nozzle, which can cause nozzle plugging resulting in incomplete expulsion of the composition.

The anti-inflammatory agent herein can have one or both of analgesic and antipyretic properties in addition to its anti-inflammatory activity. The term "anti-inflammatory agent" herein embraces compounds that are primarily analgesics or antipyretics but that have a secondary anti-inflammatory effect. Examples of anti-inflammatory agents useful herein include, but are not limited to, aceclofenac, acemetacin, e-acetamidocaproic acid, acetaminophen, acetaminosalol, acetanilide, acetylsalicylic acid (aspirin), *S*-adenosylmethionine, alclofenac, alclometasone, alfentanil, algestone, allylprodine, alminoprofen, aloxiprin, alphaprodine, aluminum bis(acetylsalicylate), amcinonide, amfenac, aminochlorthenoxazin, 3-amino-4-hydroxybutyric acid, 2-amino-4-picoline, aminopropylon, aminopyrine, amixetrine, ammonium salicylate, ampiroxicam, amtolmetin guacil, anileridine, antipyrine, antrafenine, apazone, beclomethasone, bendazac, benorylate, benoxaprofen, berizitramide, benzpiperylon, benzydamine, benzylmorphine, bermoprofen, betamethasone, bezitramide, α-bisabolol, bromfenac, p-bromoacetanilide, 5-bromosalicylic acid acetate, bromosaligenin, bucetin, bucloxic acid, bucolome, budesonide, bufexamac, bumadizon, buprenorphine, butacetin, butibufen, butophanol, carbamazepine, carbiphene, carprofen, carsalam, celecoxib, chlorobutanol, chloroprednisone, chlorthenoxazin, choline magnesium trisalicylate, choline salicylate, cinchophen, cinmetacin, cinnoxicam, ciramadol, clidanac, clobetasol, clocortolone, clometacin, clonitazene, clonixin, clopirac, cloprednol, clove, codeine, codeine methyl bromide, codeine phosphate, codeine sulfate, cortisone, cortivazol, cropropamide, crotethamide, cyclazocine, deflazacort, dehydrotestosterone, deracoxib, desomorphine, desonide, desoximetasone, dexamethasone, dexoxadrol, dextromoramide, dextropropoxyphene, dezocine, diamorphone, diampromide, diclofenac, difenamizole, difenpiramide, diflorasone, diflucortolone, diflunisal, difluprednate, dihydrocodeine, dihydrocodeinone enol acetate, dihydrocodeine phosphate, dihydromorphine, dihydroxyaluminum acetylsalicylate, dimenoxadol, dimepheptanol, dimethylthiambutene, dioxaphetyl butyrate, diphenhydramine hydrochloride, dipipanone, diprocetyl, dipyrone, ditazol, dl-chlorpheniramine maleate, droxicam, emorfazone, enfenamic acid, enoxolone, epirizole, eptazocine, etersalate, ethenzamide, ethoheptazine, etodolac, ethoxazene, ethoheptazine, ethylmethylthiambutene, ethylmorphine, etodolac, etofenamate, etonitazene, etoricoxib, eugenol, felbinac, fenbufen, fenchlofenac, fenclozic acid, fendosal, fenoprofen, fentanyl, fentiazac, fepradinol, feprazone, floctafenine, fluazacort, flucloronide, flufenamic acid, flumethasone, flunisolide, flunixin, flunoxaprofen, fluocinolone acetonide, fluocinonide, fluocinolone acetonide, fluocortin butyl, fluocortolone, fluoresone, fluorometholone, fluperolone, flupirtine, fluprednidene, fluprednisolone, fluprofen, fluproquazone, flurandrenolide, flurbiprofen, fluticasone, formocortal, fosfosal, furofenac, gentisic acid, glafenine, glucametacin, glycol salicylate, guaiazulene, halcinonide, halobetasol, halometasone, haloprednone, heroin, hydrocodone, hydrocortamate, hydrocortisone, hydromorphone, hydroxypethidine, ibufenac, ibuprofen, ibuproxam, imidazole salicylate, indomethacin, indoprofen, isofezolac, isoflupredone acetate, isoladol, isomethadone, isonixin, isoxepac, isoxicam, ketobemidone, ketoprofen, ketorolac, *p*-lactophenetide, lefetamine, levallorphan, levorphanol, levophenacyl-morphan, lofentanil, lonazolac, lornoxicam, loxoprofen, lysine acetylsalicylate, lysozyme chloride, mazipredone, meclofenamic acid, medrysone, mefenamic acid, meloxicam, meperidine, meprednisone, meptazinol, mesalamine, metazocine, methadone, methotrimeprazine, methylephedrine hydrochloride, methylprednisolone, methylsalicylate, metiazinic acid, metofoline, metopon, miroprofen, mofebutazone, mofezolac, mometasone, morazone, morphine, morphine hydrochloride, morphine sulfate, morpholine salicylate, myrophine, nabumetone, nalbuphine, nalorphine, 1-naphthyl salicylate, naproxen, narceine, nefopam, nicomorphine, nifenazone, niflumic acid, nimesulide, 5'-nitro-2'-propoxyacetanilide, norlevorphanol, normethadone, normorphine, norpipanone, noscapine, olsalazine, opium, oxaceprol, oxametacine, oxaprozin, oxipinac, oxycodone, oxymorphone, oxyphenbutazone, papaveretum, paramethasone, paranyline, parecoxib, parsalmide, pentazocine, perisoxal, phenacetin, phenadoxone, phenomorphan, phenazocine, phenazopyridine hydrochloride, phenocoll, phenoperidine, phenopyrazone, phenyl acetylsalicylate, phenylbutazone, phenylpropanolamine hydrochloride, phenyl salicylate, phenyramidol, piketoprofen, piminodine, pipebuzone, piperylone, pirazolac, piritramide, piroxicam, pirprofen, pranoprofen, prednicarbate, prednisolone, prednisone, prednival, prednylidene, proglumetacin, proheptazine, promedol, propacetamol, properidine, propiram, propoxyphene, propyphenazone, proquazone, protizinic acid, proxazole, ramifenazone, remifentanil, rimazolium metilsulfate, rofecoxib, salacetamide, salicin, salicylamide, salicylamide o-acetic acid, salicylic acid, salicylsulfuric acid, salsalate, salverine, serratiopeptidase, simetride, sudoxicam, sufentanil, sulfasalazine, sulindac, superoxide dismutase, suprofen, suxibuzone, talniflumate, tenidap, tenoxicam, terofenamate, tetrandrine, thiazolinobutazone, tiaprofenic acid, tiaprofenic acid, tiaramide, tilidine, tinoridine, tiopinac, tioxaprofen, tixocortol, tolfenamic acid, tolmetin, tramadol, triamcinolone, tropesin, valdecoxib, viminol, xenbucin, ximoprofen, zaltoprofen, zidometacin, zomepirac and the like, and combinations thereof.

In one embodiment the anti-inflammatory agent is a steroid. Suitable steroids include, but are not limited to, alclometasone, amcinonide, betamethasone, betamethasone 17-valerate, clobetasol, clobetasol propionate, clocortolone, cortisone, dehydrotestosterone, deoxycorticosterone, desonide, desoximetasone, dexamethasone, dexamethasone 21-isonicotinate, diflorasone, fluocinonide, fluocinolone, fluorometholone, flurandrenolide, fluticasone, halcinonide, halobetasol, hydrocortisone, hydrocortisone acetate, hydrocortisone cypionate, hydrocortisone hemisuccinate, hydrocortisone 21-lysinate, hydrocortisone sodium succinate, isoflupredone, isoflupredone acetate, methylprednisolone, methylprednisolone acetate, methylprednisolone sodium succinate, methylprednisolone suleptnate, mometasone, prednicarbate, prednisolone, prednisolone acetate, prednisolone hemisuccinate, prednisolone sodium phosphate, prednisolone sodium succinate, prednisolone valerate-acetate, prednisone, triamcinolone, triamcinolone acetonide and the like, and combinations thereof.

In another embodiment the anti-inflammatory agent is an analgesic, selected for example from alfentanil, allylprodine, alphaprodine, anileridine, benzylmorphine, bezitramide, buprenorphine, butorphanol, clonitazene, codeine, cyclazocine, desomorphine, dextromoramide, dextropropoxyphene, dezocine, diampromide, diamorphone, dihydrocodeine, dihydromorphine, dimenoxadol, dimepheptanol, dimethylthiambutene, dioxaphetylbutyrate, dipipanone, eptazocine, ethoheptazine, ethylmethylthiambutene, ethylmorphine, etonitazene, fentanyl, heroin, hydrocodone, hydromorphone, hydroxypethidine, isomethadone, ketobemidone, levallorphan, levorphanol, levophenacyl-morphan, lofentanil, meperidine, meptazinol, metazocine, methadone, metopon, morphine, myrophine, nalbuphine, nalorphine, narceine, nicomorphine, norlevorphanol, normethadone, normorphine, norpipanone, opium, oxycodone, oxymorphone, papaveretum, pentazocine, phenadoxone, phenazocine, phenomorphan, phenoperidine, piminodine, piritramide, proheptazine, promedol, properidine, propiram, propoxyphene, sufentanil, tilidine, tramadol and the like, and combinations thereof.

In yet another embodiment the anti-inflammatory agent is an NSAID, selected for example from salicylic acid derivatives (such as salicylic acid, acetylsalicylic acid, methyl salicylate, diflunisal, olsalazine, salsalate, sulfasalazine and the like), indole and indene acetic acids (such as indomethacin, etodolac, sulindac and the like), fenamates (such as etofenamic, meclofenamic, mefenamic, flufenamic, niflumic and tolfenamic acids and the like), heteroaryl acetic acids (such as acemetacin, alclofenac, clidanac, diclofenac, fenchlofenac, fentiazac, furofenac, ibufenac, isoxepac, ketorolac, oxipinac, tiopinac, tolmetin, zidometacin, zomepirac and the like), aryl acetic acid and propionic acid derivatives (such as alminoprofen, benoxaprofen, bucloxic acid, carprofen, fenbufen, fenoprofen, fluprofen, flurbiprofen, ibuprofen, indoprofen, ketoprofen, miroprofen, naproxen, naproxen sodium, oxaprozin, pirprofen, pranoprofen, suprofen, tiaprofenic acid, tioxaprofen and the like), enolic acids (such as the oxicam derivatives ampiroxicam, cinnoxicam, droxicam, lornoxicam, meloxicam, piroxicam, sudoxicam and tenoxicam, and the pyrazolone derivatives aminopyrine, antipyrine, apazone, dipyrone, oxyphenbutazone, phenylbutazone and the like), para-aminophenol derivatives (such as acetaminophen and the like), alkanones (such as nabumetone and the like), nimesulide, proquazone and the like, and combinations thereof.

In a preferred embodiment the anti-inflammatory agent is a selective COX-2 inhibitor. A selective COX-2 inhibitor is a compound that selectively inhibits cyclooxygenase-2 (COX-2) activity. The terms "selective COX-2 inhibitor" and "selective cyclooxygenase-2 inhibitor" interchangeably refer to a therapeutic compound that selectively inhibits the COX-2 isoform of the enzyme cyclooxygenase, with less significant inhibition of cyclooxygenase-1 (COX-1). As used herein the term "selective COX-2 inhibitor" also refers to a prodrug or salt that is converted *in vivo* to a compound that exhibits selective inhibition of COX-2 relative to COX-1. Preferred selective COX-2 inhibitors exhibit a selectivity factor of at least about 10, more preferably at least about 50 and still more preferably at least about 100, wherein "selectivity factor" is defined as IC₅₀ (COX-1)/IC₅₀(COX-2), IC₅₀ being the concentration of a compound producing 50% inhibition of enzyme activity in an *in vitro* or *in vivo* test.

Selective COX-2 inhibitors applicable to the invention include, but are not limited to, the compounds described below and include tautomers, stereoisomers, enantiomers, salts, hydrates, prodrugs and combinations thereof. Any such selective COX-2 inhibitory drug or prodrug known in the art can be used.

A preferred selective COX-2 inhibitory drug useful herein is a compound of formula (I): 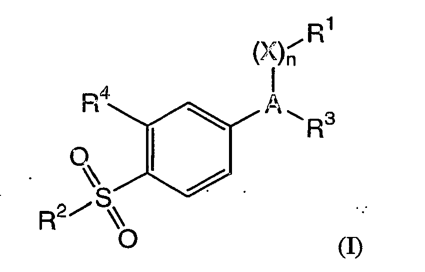 or a prodrug or pharmaceutically acceptable salt thereof, wherein:
- A is: a substituent selected from partially unsaturated or unsaturated heterocyclyl and partially unsaturated or unsaturated carbocyclic rings, preferably a heterocyclyl group selected from pyrazolyl, furanonyl, isoxazolyl, pyridinyl, cyclopentenonyl and pyridazinonyl groups;
- X is: O, S or CH₂;
- n is: 0 or 1;
- R¹ is: at least one substituent selected from heterocyclyl, cycloalkyl, cycloalkenyl and aryl, and is optionally substituted at a substitutable position with one or more radicals selected from alkyl, haloalkyl, cyano, carboxyl, alkoxycarbonyl, hydroxyl, hydroxyalkyl, haloalkoxy, amino, alkylamino, arylamino, nitro, alkoxyalkyl, alkylsulfinyl, halo, alkoxy and alkylthio;
- R² is: methyl, amino or aminocarbonylalkyl;
- R³ is: one or more radicals selected from hydrido, halo, alkyl, alkenyl, alkynyl, oxo, cyano, carboxyl, cyanoalkyl, heterocyclyloxy, alkyloxy, alkylthio, alkylcarbonyl, cycloalkyl, aryl, haloalkyl, heterocyclyl, cycloalkenyl, aralkyl, heterocyclylalkyl, acyl, alkylthioalkyl, hydroxyalkyl, alkoxycarbonyl, arylcarbonyl, aralkylcarbonyl, aralkenyl, alkoxyalkyl, arylthioalkyl, aryloxyalkyl, aralkylthioalkyl, aralkoxyalkyl, alkoxyaralkoxyalkyl, alkoxycarbonylalkyl, aminocarbonyl, aminocarbonylalkyl, alkylaminocarbonyl, N-arylaminocarbonyl, N-alkyl-N-arylaminocarbonyl, alkylaminocarbonylalkyl, carboxyalkyl, alkylamino, N-arylamino, N-aralkylamino, N-alkyl-N-aralkylamino, N-alkyl-N-arylamino, aminoalkyl, alkylaminoalkyl, N-arylaminoalkyl, N-aralkylaminoalkyl,N-alkyl-N-aralkylaminoalkyl, N-alkyl-N-arylaminoalkyl, aryloxy, aralkoxy, arylthio, aralkylthio, alkylsulfinyl, alkylsulfonyl, aminosulfonyl, alkylaminosulfonyl, N-arylaminosulfonyl, arylsulfonyl and N-alkyl-N-arylaminosulfonyl, R³ being optionally substituted at a substitutable position with one or more radicals selected from alkyl, haloalkyl, cyano, carboxyl, alkoxycarbonyl, hydroxyl, hydroxyalkyl, haloalkoxy, amino, alkylamino, arylamino, nitro, alkoxyalkyl, alkylsulfinyl, halo, alkoxy and alkylthio; and
- R⁴ is: selected from hydrido and halo.

A particularly preferred group of selective COX-2 inhibitory drugs are compounds having the formula (II): 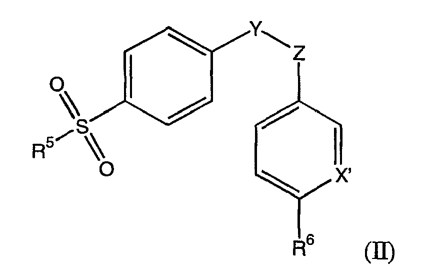 where R⁵ is a methyl or amino group, R⁶ is hydrogen or a C₁₋₄ alkyl or alkoxy group, X' is N or CR⁷ where R⁷ is hydrogen or halogen, and Y and Z are independently carbon or nitrogen atoms defining adjacent atoms of a five- to six-membered ring that is optionally substituted at one or more positions with oxo, halo, methyl or halomethyl groups, or an isomer, tautomer, pharmaceutically-acceptable salt or prodrug thereof. Preferred such five- to six-membered rings are cyclopentenone, furanone, methylpyrazole, isoxazole and pyridine rings substituted at no more than one position.

Another particularly preferred group of selective COX-2 inhibitory drugs are compounds having the formula (III): 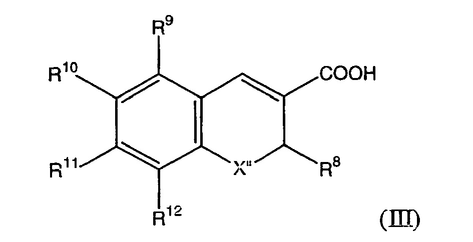 where X" is O, S or N-lower alkyl; R⁸ is lower haloalkyl; R⁹ is hydrogen or halogen; R¹⁰ is hydrogen, halogen, lower alkyl, lower alkoxy or haloalkoxy, lower aralkylcarbonyl, lower dialkylaminosulfonyl, lower alkylaminosulfonyl, lower aralkylaminosulfonyl, lower heteroaralkylaminosulfonyl, or 5- or 6- membered nitrogen-containing heterocyclosulfonyl; and R¹¹ and R¹² are independently hydrogen, halogen, lower alkyl, lower alkoxy or aryl; and pharmaceutically acceptable salts thereof.

A particularly useful compound of formula (III) is (S)-6,8-dichloro-2-(trifluoromethyl)-2H-1-benzopyran-3-carboxylic acid.

Another particularly preferred group of selective COX-2 inhibitory drugs are 5-alkyl-2-arylaminophenylacetic acids and derivatives thereof. Particularly useful compounds of this class are lumiracoxib and pharmaceutically acceptable salts thereof.

Illustratively, celecoxib, deracoxib, valdecoxib, parecoxib, rofecoxib, etoricoxib, lumiracoxib, 2-(3,5-difluorophenyl)-3-[4-(methylsulfonyl)phenyl]-2-cyclopenten-1-one, (S)-6,8-dichloro-2-(trifluoromethyl)-2H-1-benzopyran-3-carboxylic acid, 2-(3,4-difluorophenyl)-4-(3-hydroxy-3-methyl-1-butoxy)-5-[4-(methylsulfonyl)phenyl]-3-(2H)-pyridazinone, 4-[5-(4-fluorophenyl)-3-(trifluoromethyl)-1H-pyrazol-1-yl]benzenesulfonamide, 4-[5-(phenyl)-3-(trifluoromethyl)-1H-pyrazol-1-yl]benzenesulfonamide and their salts, more particularly celecoxib, deracoxib, valdecoxib, parecoxib and its salts, rofecoxib, etoricoxib, lumiracoxib, 4-[5-(4-fluorophenyl)-3-(trifluoromethyl)-1H-pyrazol-1-yl]benzenesulfonamide and 4-[5-(phenyl)-3-(trifluoromethyl)-1H-pyrazol-1-yl]benzenesulfonamide are useful in the method and composition of the invention.

Valdecoxib used in compositions of the invention can be prepared by any known process, for example in the manner set forth in U.S. Patent No. 5,633,272 to Talley *et al.* Parecoxib and salts thereof used in compositions of the invention can be prepared by any known process, for example in the manner set forth in U.S. Patent No. 5,932,598 to Talley *et al.* Rofecoxib used in compositions of the invention can be prepared by any known process, for example in the manner set forth in U.S. Patent No. 5,474,995 to Ducharme *et al.* Etoricoxib used in compositions of the invention can be prepared by any known process, for example in the manner set forth in International Patent Publication No. WO 98/03484. 2-(3,5-Difluorophenyl)-3-[4-(methylsulfonyl) phenyl]-2-cyclopenten-1-one used in compositions of the invention can be prepared by any known process, for example in the manner set forth in European Patent No. 0 863 134. Deracoxib used in compositions of the invention can be prepared by any known process, for example in the manner set forth in U.S. Patent No. 5,466,823 to Talley *et al.*2-(3,4-Difluorophenyl)-4-(3-hydroxy-3-methyl-1-butoxy)-5-[4-(methylsulfonyl)phenyl]-3-(2H)-pyridazinone used in compositions of the invention can be prepared by any known process, for example in the manner set forth in International Patent Publication No. WO 00/24719. Other selective COX-2 inhibitory drugs can be prepared by any known process, including processes set forth in patent publications disclosing such drugs; for example in the case of celecoxib in above-cited U.S. Patent No. 5,466,823 or in U.S. Patent No. 5,892,053 to Zhi et al.

Where the anti-inflammatory agent is a selective COX-2 inhibitor a preferred concentration range in a composition of the invention is about 0.01 to about 1000 mg/ml, more preferably about 0.1 to about 750 mg/ml, and still more preferably about 5 to about 250 mg/ml. For second agents other than a selective COX-2 inhibitor, suitable concentration ranges can be determined by one of skill in the art based upon published data.

It should be understood that any reference herein to a particular drug compound includes tautomers, stereoisomers, enantiomers, salts, hydrates and prodrugs of that compound and is not specific to any one solid state form of the drug unless the context so requires.

In one embodiment the second agent administered in combination therapy with the selective COX-2 inhibitor is an antibacterial agent. Antibacterial agents applicable for use according to the invention include any such agents that are effective for treatment and/or prevention of mammary disorders and/or otic disorders and/or complications associated therewith. Suitable antibacterial agents include, but are not limited to, beta-lactam antibacterials such as natural and synthetic penicillin type agents including penam penicillins (such as benzyl penicillin, phenoxymethyl penicillin, coxacillin, nafcillin, methicillin, oxacillin, amoxycillin, temocillin, ticarcillin and the like), penicillinase-stable penicillins, acylamino and carboxypenicillins (such as piperacillin, azlocillin, mezlocillin, carbenicillin, temocillin, ticarcillin and the like), and broader spectrum penicillins (such as streptomycin, neomycin, framycetin, gentamicin, apramycin, amikacin, spectinomycin, amoxycillin, ampicillin and the like), cephalosporins, macrolides (such as tylosin, tilmicosin, aivlosin, erythromycin, azithromycin, spiramycin, josamycin, kitasamycin and the like), lincosamides (such as lincomycin, clindamycin, pirlimycin and the like), pleuromutilins (such as tiamulin, valnemulin and the like); polypeptides, glycopeptides (such as vancomycin and the like), polymixins (such as polymixin B, polymixin E and the like), sulfonamides (such as sulfamethazine, sulfadiazine, silver sulfadiazine, sulfatroxazole, sulfamethoxypyridazine, sulfanilamide, sulfamethoxazole, sulfisoxazole, sulfamethizole, mafenide and the like, alone or in combination with trimethoprim), chloramphenicol, thiamphenicol, florfenicol, tetracycline type agents (such as tetracycline, chlortetracycline, oxytetracycline, domeclocycline, doxycycline, minocycline and the like), quinolones and fluoroquinolones (such as ciprofloxacin, enoxacin, grepafloxacin, levofloxacin, lomefloxacin, norfloxacin, ofloxacin, sparfloxacin, trovafloxacin, cinocacin, nalidixic acid and the like), tiamulin, colistin, meropenem, sulbactam, tazobactam, methacycline, pyrimethamine, sulfacetamide, oxazolidinones, *e.g*., eperezolid, linezolid, N-((5S)-3-(3-fluoro-4-(4-(2-fluoroethyl)-3-oxy-1-piperazinyl)phenyl-2-oxy-5-oxazolidinyl)methyl)acetamide, (S)-N-((3-(5-(3- pyridyl)thiophen-2-yl)-2-oxy-5-oxazolidinyl)methyl)acetamide, 2,2-difluoro-*N*-({(5*S*)-3-[3-fluoro-4-(4-glycoloylpiperazin-1-yl)phenyl]-2-oxo-1,3-oxazolidin-5-yl}methyl)ethanethioamide, (S)-N-((3-(5-(4-pyridyl)pyrid-2-yl)-2-oxy-5-oxazolidinyl)methyl)acetamide hydrochloride and the like, aminoglycosides (kanamycin, tobramycin, netilmicin and the like), aminocyclitols, amphenicol, ansamycin, carbaphenem, cephamycin, rifampicin, monobactam, oxacephem, streptogramins (such as quinupristin, dalfopristin and the like), cycloserines, mupirocin, urea hydroxamates, folic acid analogs (such as trimethoprim and the like), antibiotic-type antineoplastic agents (such as aclarubicin, actinomycin D, actinoplanone, aeroplysinin derivative, Nippon Soda anisomycins, anthracycline, azino-micyin-A, busucaberin, bleomycin sulfate, bryostatin-1, calichemycin, chromoximycin, dactinomycin, daunorubicin, ditrisarubicin B, doxorubicin, doxorubicin-fibrinogen, elsamicin-A, epirubicin, erbstatin, esorubicin, esperamicin-A1b, fostriecin, glidobactin, gregatin-A, grincamycin, herbimycin, idarubicin, illudins, kazusamycin, kesarirhodins, menogaril, mitomycin, mitoxantorone, mutamycin, mycophenolate mofetil, neoenactin, oxalysine, oxaunomycin, peplomycin, pilatin, pirarubicin, porothramycin, pyrindamycin A, rapamycin, rhizoxin, rodorubicin, sibanomicin, siwenmycin, sorangicin-A, sparsomycin, steffimycin B, talisomycin, terpentecin, thrazine, tricrozarin A, zorubicin, systemic antibacterials (such as 2,4-diaminopyrimidine), nitrofuran sulfones, marbofloxacin and the like, and combinations thereof

Preferred antibacterial agents are cephalosporins including, but not limited to, ceftiofur hydrochloride, ceftiofur free acid, *e.g*., ceftiofur crystalline free acid, ceftiofur sodium, other ceftiofur salts, cephalexin, cephradine, cefquinome, cephacetrile, cephalonium, cefuroxime, cefazidime, cefoperazone, sodium cephemethcarboxylate, cephem heptahydrate, cephalosporin di- or tri-hydrate, cephadroxil monohydrate, cephazolin sodium monohydrate, cefiximine, ceftaxime, ceftizoxime, ceftriaxone, o-formylcefamandole, salts of 3-acetoxymethyl-7-(iminocetamido)-cephalosporanic acid derivatives, monohydrate of 7-(D-alpha-amino-alpha-(p-hydroxyphenyl)acetamino)-3-methyl-3-cephem-1-carboxylic acid, hydrochloride salt of syn-7-((2-amino-1-thiazolyl)(methoxyimino)acetyl)amino)-3-methyl-3-cephem-4-carboxylic acid, cephem acid addition salts, (pivaloyloxy)methyl 7-beta-(2-(2-amino-4-thiazolyl)acetamido)-3-(((1-(2-(dimethylamino)ethyl)-1H-tetraazol-5-yl)thio)methyl)-3-cephem-4-carboxylate, cephalexin, cephalexin monohydrate, 7-(D-2-naphthyglycylamino)-3-methyl-3-cephem-4-carboxylic acid tetrahydrate and the like. The most preferred cephalosporins for use according to the present invention are ceftiofur and pharmaceutically acceptable salts thereof. Especially preferred are ceftiofur free acid, most especially in crystalline form, and ceftiofur hydrochloride.

Where the antibacterial substance is ceftiofur or a salt thereof, a preferred concentration range in a composition of the invention is about 1 to about 1000 mg/ml, more preferably about 5 to about 750 mg/ml, and still more preferably about 10 to about 100 mg/ml. For antibacterial substances other than ceftiofur, suitable concentration ranges that are antibacterially equivalent can be determined by one of skill in the art based upon published data.

In another embodiment the second agent is an antineoplastic agent. Suitable antineoplastic agents include, but are not limited to, anastrozole, calcium carbonate, capecitabine, carboplatin, cisplatin, docetaxel, eflornithine, etoposide, exemestane, fluoxymestrine, gemcitabine, goserelin, irinotecan, ketoconazole, letrozol, leucovorin, levamisole, megsetrol, paclitaxel, raloxifene, retinoic acid, selenium (selenomethionine), sulindac sulfone, tamoxifen, thiotepa, topotecan, toremifen, vinbastine, vincristin, vinorelbine and the like, and combinations thereof.

Amphipathic oils applicable to the current invention include all amphipathic oils that are water dispersible and ethanol insoluble.

Preferred such amphipathic oils are polyglycolized glycerides prepared by an alcoholosis reaction of natural triglycerides with polyethylene glycols, and examples include, but are not limited to, the following Gattefossé oils or substantially equivalent oils from another manufacturer: Labrafil^{™} M-1944CS, Labrafil^{™} M-1966CS, Labrafil^{™} M-1969CS, Labrafil^{™} M-1980CS, Labrafil^{™} M-2125CS, Labrafil^{™} WL-2609BS, Labrafil^{™} ISO and combinations thereof.

Still more preferred amphipathic oils are polyglycolized glycerides prepared as above, comprising a main fatty acid component of either oleic acid or linoleic acid, and examples include, but are not limited to, the following Gattefossé oils or substantially equivalent oils from another manufacturer: Labrafil^{™} M-1944CS, Labrafil^{™} M-1966CS, Labrafil^{™} M-1969CS, Labrafil^{™} M-1980CS, Labrafil^{™} M-2125CS, Labrafil^{™} WL-2609BS and combinations thereof.

Still more preferred amphipathic oils are polyglycolized glycerides prepared as above, comprising a main fatty acid component of oleic acid, and examples include, but are not limited to, the following Gattefossé oils or substantially equivalent oils from another manufacturer: Labrafil^{™} M-1944CS, Labrafil^{™} M-1966CS, Labrafil^{™} M-1980CS and combinations thereof.

The most preferred amphipathic oil is pegicol 5-oleate, for example Labrafil^{™} M-1944CS of Gattfossé Corporation.

A preferred concentration range for the amphipathic oil in a composition of the invention is about 0.01% to about 99% weight/volume, more preferably about 1% to about 80% weight/volume, and still more preferably about 3% to about 25% weight/volume.

Microcrystalline wax is as defined for example in *Handbook of Pharmaceutical Excipients,* 3rd ed. or in *National Formulary,* 19th ed. (NF 19) and can be obtained from a number of manufacturers including Witco Corporation.

A preferred concentration range for microcrystalline wax in a composition of the invention is about 0.001% to about 50% weight/volume, more preferably about 0.1 % to about 40% weight/volume, and still more preferably about 1% to about 15% weight/volume.

Pharmaceutically acceptable non-aqueous carriers of the invention can be fully saturated, or partially or fully unsaturated. Examples of non-aqueous carriers include, but are not limited to, vegetable oils, mineral oils, synthetic oils and combinations thereof. Examples of fully saturated non-aqueous carriers include, but are not limited to, esters of medium to long chain fatty acids (such as fatty acid triglycerides with a chain length of about C₆ to about C₂₄). Mixtures of fatty acids are split from the natural oil (for example coconut oil, palm kernel oil, babassu oil or the like) and are refined. In some embodiments, medium chain (about C₈ to about C₁₂) triglycerides are useful. An illustrative saturated non-aqueous carrier comprises capric acid (about 20% to about 45%) and caprylic acid (about 45% to about 80%). Other fully saturated non-aqueous carriers include, but are not limited to, saturated coconut oil (which typically includes a mixture of lauric, myristic, palmitic, capric and caproic acids), including those sold under the Miglyol^{™} trademark from Huls and bearing trade designations 810, 812, 829 and 840). Also noted are the NeoBee^{™} products sold by Drew Chemicals. Isopropyl myristate is another example of a non-aqueous carrier useful in compositions of the invention. Examples of synthetic oils include triglycerides and propylene glycol diesters of saturated or unsaturated fatty acids having 6 to 24 carbon atoms such as, for example hexanoic acid, octanoic (caprylic), nonanoic (pelargonic), decanoic (capric), undecanoic, lauric, tridecanoic, tetradecanoic (myristic), pentadecanoic, hexadecanoic (palmitic), heptadecanoic, octadecanoic (stearic), nonadecanoic, heptadecanoic, eicosanoic, heneicosanoic, docosanoic and lignoceric acids and the like. Examples of unsaturated carboxylic acids include oleic, linoleic and linolenic acids and the like. It is understood that the non-aqueous carrier can comprise the mono-, di- and triglyceryl esters of fatty acids or mixed glycerides and/or propylene glycol diesters wherein at least one molecule of glycerol has been esterified with fatty acids of varying carbon atom length. A nonlimiting example of a "non-oil" useful as a carrier in compositions of the invention is polyethylene glycol.

Preferred non-aqueous carriers are vegetable oils such as cottonseed oil, corn oil, sesame oil, soybean oil, olive oil, fractionated coconut oil, peanut oil, sunflower oil, safflower oil, almond oil, avocado oil, palm oil, palm kernel oil, babassu oil, beechnut oil, linseed oil, rape oil and the like. The most preferred non-aqueous carrier is cottonseed oil. By way of example cottonseed oil is available in a preparation of 70% unsaturated fatty acids from Sigma Chemical Co.

A preferred concentration range for the non-aqueous carrier in a composition of the invention is about 0.5% to about 99% weight/volume, more preferably about 10% to about 95% weight/volume, and still more preferably about 40% to about 90% weight/volume.

A composition of the invention can optionally further comprise any conventional pharmaceutical excipient that does not deleteriously react with the essential ingredients of the composition. Such excipients include, but are not limited to, antioxidants, preservatives, suspending agents, stabilizers, solubilization agents, wetting agents, lubricants, emulsifiers, salts for influencing osmotic pressure, coloring agents, alcohols, isotonic agents, buffering agents and combinations thereof.

A composition of the invention can be administered for treatment of mastitis by inserting the cannula nozzle of a mastitis syringe into the external orifice of the teat canal of an udder of a milk producing animal and infusing the composition into the udder.

A composition of the invention can be administered for treatment or prevention of an otic disorder by inserting the nozzle of an ear syringe, otic drop dispenser or other appropriate otic delivery device into the external auditory canal of the ear of a subject and infusing the composition into the ear.

It will be appreciated that preferred amounts of compositions to be administered in a specific case will vary according to the specific composition being utilized, the mode of application, the particular situs and organism being treated, and other factors. Dosages for a given purpose can be determined using conventional considerations, for example, by customary comparison of the differential activities of the subject compositions and of a known agent, *e.g*., by means of an appropriate conventional pharmaceutical protocol.

Although the present invention is directed primarily to local delivery of an anti-inflammatory agent to a site of inflammation in the organ to which it is administered, it is contemplated that compositions of the invention are also useful for systemic delivery of the anti-inflammatory agent to a milk producing animal via intramammary infusion. For example, it is often more efficient and convenient to administer a therapeutic agent to a milk producing animal such as a cow by intramammary infusion than by other routes, such as orally or parenterally. Thus in treatment of inflammatory conditions elsewhere than the udder, including for example arthritic conditions, a composition as herein described can be administered by intramammary infusion.

An illustrative suspension composition of the invention containing an anti-inflammatory agent, *e.g*., the selective COX-2 inhibitor deracoxib, has the following composition:

| | |
|---|---|
| selective COX-2 inhibitor | 1-350 mg/ml |
| Labrafil^{™} M-1944CS | 1-75% |
| microcrystalline wax | 0.1-25% |
| cottonseed oil | *q.s*. to 100% |

### EXAMPLES

The following examples illustrate aspects of the present invention.

### Example 1

A suspension to be administered by intramammary infusion is prepared having the following composition:

| | |
|---|---|
| parecoxib free acid | 100 mg/ml |
| Labrafil^{™} M-1944CS | 50 mg/ml |
| microcrystalline wax NF | 70 mg/ml |
| cottonseed oil NF | *q.s.* |

The microcrystalline wax and approximately 27% of the total amount of the cottonseed oil are heated to 85-98°C with mixing, in a kettle. The balance of the cottonseed oil is heated to 85-98°C with mixing, in a manufacturing tank. After the microcrystalline wax is completely melted the microcrystalline wax/cottonseed oil mixture in the kettle is transferred to the manufacturing tank containing cottonseed oil and mixed thoroughly. The resulting mixture is cooled to 38-45°C and the Labrafil^{™} M-1944CS is added to the manufacturing tank with mixing to form a vehicle. The parecoxib is then added to the vehicle and the resulting composition is mixed to form a unifonn suspension. The suspension is screened and filled into 12 ml high density polyethylene mastitis syringes. The packaged product is terminally sterilized by gamma irradiation at a dose of 25-40 kGy.

The above suspension is administered by intramammary infusion to each infected quarter of an udder of a lactating cow at a dose of 1,200 mg parecoxib/quarter/day. The suspension is effective in treatment of lactating cow mastitis.

### Example 2

A suspension to be administered by intramammary infusion is prepared having the following composition:

| | |
|---|---|
| deracoxib | 170 mg/ml |
| Labrafil^{™} M-1966CS | 100 mg/ml |
| microcrystalline wax NF | 50 mg/ml |
| corn oil NF | *q.s.* |

The microcrystalline wax and the corn oil are heated to 85-98°C with mixing, in a manufacturing tank. After the microcrystalline wax is completely melted, the mixture is cooled to 30-45°C and the Labrafil^{™} M-1966CS is added to the manufacturing tank with mixing to form a vehicle. The deracoxib is added to the vehicle and mixed to form a uniform suspension. The suspension is screened and filled into 12 ml high density polyethylene mastitis syringes. The packaged product is terminally sterilized by gamma irradiation at a dose of 25-40 kGy.

The above suspension is administered to all four quarters of a dry cow at a dose of 3,400 mg deracoxib/quarter by intramammary infusion. The suspension is effective in treatment of dry cow mastitis.

### Example 3

A suspension to be administered by otic infusion is prepared having the following composition:

| | |
|---|---|
| rofecoxib | 25 mg/ml |
| Labrafil^{™} M-1980CS | 500 mg/ml |
| microcrystalline wax NF | 0.10 mg/ml |
| propyl gallate | 1.0 mg/ml |
| mineral oil | *q.s*. |

The microcrystalline wax and approximately 27% of the total amount of mineral oil are heated to 85-98°C with mixing, in a kettle. The balance of the mineral oil is heated to 85-98°C with mixing, in a manufacturing tank. After the microcrystalline wax is completely melted, the microcrystalline wax/mineral oil mixture in the kettle is transferred to the manufacturing tank containing mineral oil and mixed thoroughly. The resulting mixture is cooled to 38-45°C and the Labrafil^{™} M-1980CS is added to the manufacturing tank with mixing. The propyl gallate is added to the manufacturing tank with mixing to form the vehicle. The rofecoxib is added to the resulting vehicle and mixed to form a uniform suspension. The suspension is screened and filled into 20 ml polypropylene containers.

The above suspension is administered at a dose of 2.5 mg rofecoxib/kg of body weight, by infusion to the ear of a dog. The suspension is effective in treatment of canine otitis externa.

### Example 4

A suspension to be administered by intramammary infusion is prepared having the following composition:

| | |
|---|---|
| deracoxib | 300 mg/ml |
| Labrafil^{™} M-1944CS | 50 mg/ml |
| microcrystalline wax NF | 70 mg/ml |
| cottonseed oil NF | *q.s.* |

The microcrystalline wax and approximately 27% of the total amount of the cottonseed oil are heated to 85-98°C with mixing, in a kettle. The balance of the cottonseed oil is heated to 85-98°C with mixing, in a manufacturing tank. After the microcrystalline wax is completely melted, the microcrystalline wax/cottonseed oil mixture in the kettle is transferred to the manufacturing tank containing cottonseed oil and mixed thoroughly. The resulting mixture is cooled to 38-45°C and the Labrafil^{™} M-1944CS is added to the manufacturing tank with mixing to form the vehicle. The deracoxib is added to the resulting vehicle and mixed to form a uniform suspension. The suspension is screened and filled into 12 ml high density polyethylene mastitis syringes. The packaged product is terminally sterilized by gamma irradiation at a dose of 25-40 kGy.

The above suspension is administered to all four quarters of a dry cow at a dose of 12,000 mg deracoxib/quarter by intramammary infusion. The suspension is effective in treatment of dry cow mastitis.

### Example 5

A suspension to be administered by intramammary infusion is prepared having the following composition:

| | |
|---|---|
| valdecoxib | 1.5 mg/ml |
| Labrafil^{™} WL-2609BS | 75 mg/ml |
| microcrystalline wax NF | 100 mg/ml |
| Miglyol^{™} 812 | *q.s.* |

The microcrystalline wax and approximately 30% of the total amount of the Miglyol^{™} 812 are heated to 85-98°C with mixing, in a kettle. The balance of the Miglyol^{™} 812 is heated to 85-98°C with mixing, in a manufacturing tank. After the microcrystalline wax is completely melted, the microcrystalline wax/Miglyol^{™} 812 mixture in the kettle is transferred to the manufacturing tank containing the Miglyol^{™} 812 and mixed thoroughly. The resulting mixture is cooled to 38-45°C and the Labrafil^{™} WL-2609BS is added to the manufacturing tank with mixing to form the vehicle. The valdecoxib is added to the resulting vehicle and mixed to form a uniform suspension. The suspension is screened and filled into 12 ml high density polyethylene mastitis syringes. The packaged product is terminally sterilized by gamma irradiation at a dose of 25-40 kGy.

The above suspension is administered to all four quarters of a dry cow at a dose of 30 mg valdecoxib/quarter by intramammary infusion. The suspension is effective in treatment of dry cow mastitis.

### Example 6

A suspension to be administered by otic infusion is prepared having the following composition:

| | |
|---|---|
| ceftiofur hydrochloride (micronized) | 100 mg/ml |
| deracoxib | 100 mg/ml |
| Labrafil^{™} M-1944CS | 700 mg/ml |
| microcrystalline wax NF | 0.05 mg/ml |
| mineral oil | *q.s.* |

The microcrystalline wax and approximately 27% of the total amount of mineral oil are heated to 85-98°C with mixing, in a kettle. The balance of the mineral oil is heated to 85-98°C with mixing, in a manufacturing tank. After the microcrystalline wax is completely melted, the microcrystalline wax/mineral oil mixture in the kettle is transferred to the manufacturing tank containing mineral oil and mixed thoroughly. The resulting mixture is cooled to 38-45°C and the Labrafil^{™} M-1944CS is added to the manufacturing tank with mixing to form the vehicle. The ceftiofur hydrochloride and the deracoxib are added to the resulting vehicle and mixed to form a uniform suspension. The suspension is screened and filled into 50 ml polypropylene containers.

The above suspension is administered at a dose of 4 mg ceftiofur hydrochloride/kg body weight and 4 mg deracoxib/kg of body weight by infusion to the ear of a subject. The suspension is effective in treatment and/or prevention of otitis media.

### Example 7

A suspension to be administered by otic infusion is prepared having the following composition:

| | |
|---|---|
| ceftiofur hydrochloride (micronized) | 100 mg/ml |
| parecoxib free acid | 100 mg/ml |
| Labrafil^{™} M-1944CS | 700 mg/ml |
| microcrystalline wax NF | 0.1 mg/ml |
| cottonseed oil NF | *q.s.* |

The microcrystalline wax and cottonseed oil are heated to 85-98°C with mixing, in a manufacturing tank. After the microcrystalline wax is completely melted, the mixture is cooled to 38-45°C and the Labrafil^{™} M-1944CS is added to the manufacturing tank with mixing to form the vehicle. The ceftiofur hydrochloride and parecoxib are added to the resulting vehicle and mixed to form a uniform suspension. The suspension is screened and filled into 60 ml polypropylene containers.

The above suspension is administered at a dose of 4 mg ceftiofur hydrochloride/kg body weight and 4 mg parecoxib/kg of body weight by infusion into the ear of a subject. The combination therapy is effective in treatment and/or prevention of otitis externa.

## Claims

1. Use of a pharmaceutical composition comprising an anti-inflammatory agent for the manufacture of a medicament for the treatment and/or prevention of an inflammatory condition in a fluid-containing organ having a natural exterior orifice by administering the composition to the organ via the exterior orifice, said composition further comprising a vehicle that comprises (a) an amphipathic oil that is water dispersible and ethanol insoluble, (b) microcrystalline wax and (c) a pharmaceutically acceptable non-aqueous carrier.

2. The use of Claim 1 wherein the fluid-containing organ is an udder of a milk producing animal, and wherein the composition is administered by intramammary infusion.

3. The use of Claim 2 wherein the inflammatory condition is associated with mastitis.

4. The use of Claim 1 wherein the fluid-containing organ is an ear of a subject, and wherein the composition is administered by infusion into the ear.

5. The use of Claim 4 wherein the inflammatory condition is associated with an otic disorder selected from the group consisting of otitis externa, otitis media, otorrhea, acute mastoiditis, infections related to otic surgical procedures, otosclerosis, otalgia, otic pain, otic inflammation, otic bleeding, Lermoyez's syndrome, Meniere's disease, vestibular neuronitis, benign paroxysmal positional vertigo, herpes zoster oticus, Ramsay Hunt's syndrome, viral neuronitis, ganglionitis, geniculate herpes, labyrinthitis, including purulent labyrinthitis and viral endolymphatic labyrinthitis, perilymph fistulas, presbycusis, drug-induced ototoxicity, acoustic neuromas, aerotitis media, infectious myringitis, bullous myringitis, otic neoplasm, squamous cell carcinoma, basal cell carcinoma, other otic cancers, pre-cancerous otic conditions, nonchromaffin paragangliomas, chemodectomas, glomus jugulare tumors, glomus tympanicum tumors, perichondritis, aural eczematoid dermatitis, malignant external otitis, subperichondrial hematoma, ceruminomas, impacted cerumen, sebaceous cysts, osteomas, keloids, tinnitus, vertigo, tympanic membrane infection, tympanitis, otic furuncles, petrositis, conductive and sensorineural hearing loss, epidural abscess, lateral sinus thrombosis, subdural empyema, otitic hydrocephalus, Dandy's syndrome, bullous myringitis, diffuse external otitis, foreign bodies, keratosis obturans, otomycosis, trauma, acute barotitis media, acute eustachian tube obstruction, postsurgical otalgia, and complications associated therewith.

6. The use of Claim 4 wherein the inflammatory condition is associated with an otic disorder selected from the group consisting of otitis externa, otitis media, otorrhea, and infections having an inflammatory component that are related to an otic surgical procedure.

7. The use of Claim 1 wherein the anti-inflammatory agent is selected from the group consisting of aceclofenac, acemetacin, e-acetamidocaproic acid, acetaminophen, acetaminosalol, acetanilide, acetylsalicylic acid, *S*-adenosylmethionine, alclofenac, alclometasone, alfentanil, algestone, allylprodine, alminoprofen, aloxiprin, alphaprodine, aluminum bis(acetylsalicylate), amcinonide, amfenac, aminochlorthenoxazin, 3-amino-4-hydroxybutyric acid, 2-amino-4-picoline, aminopropylon, aminopyrine, amixetrine, ammonium salicylate, ampiroxicam, amtolmetin guacil, anileridine, antipyrine, antrafenine, apazone, beclomethasone, bendazac, benorylate, benoxaprofen, benzpiperylon, benzydamine, benzylmorphine, bermoprofen, betamethasone, betamethasone-17-valerate, bezitramide, α-bisabolol, bromfenac, *p*-bromoacetanilide, 5-bromosalicylic acid acetate, bromosaligenin, bucetin, bucloxic acid, bucolome, budesonide, bufexamac, bumadizon, buprenorphine, butacetin, butibufen, butorphanol, carbamazepine, carbiphene, carprofen, carsalam, chlorobutanol, chloroprednisone, chlorthenoxazin, choline salicylate, cinchophen, cinmetacin, ciramadol, clidanac, clobetasol, clocortolone, clometacin, clonitazene, clonixin, clopirac, cloprednol, clove, codeine, codeine methyl bromide, codeine phosphate, codeine sulfate, cortisone, cortivazol, cropropamide, crotethamide, cyclazocine, deflazacort, dehydrotestosterone, desomorphine, desonide, desoximetasone, dexamethasone, dexamethasone-21-isonicotinate, dexoxadrol, dextromoramide, dextropropoxyphene, deoxycorticosterone, dezocine, diampromide, diamorphone, diclofenac, difenamizole, difenpiramide, diflorasone, diflucortolone, diflunisal, difluprednate, dihydrocodeine, dihydrocodeinone enol acetate, dihydromorphine, dihydroxyaluminum acetylsalicylate, dimenoxadol, dimepheptanol, dimethylthiambutene, dioxaphetyl butyrate, dipipanone, diprocetyl, dipyrone, ditazol, droxicam, emorfazone, enfenamic acid, enoxolone, epirizole, eptazocine, etersalate, ethenzamide, ethoheptazine, ethoxazene, ethylmethylthiambutene, ethylmorphine, etodolac, etofenamate, etonitazene, eugenol, felbinac, fenbufen, fenclozic acid, fendosal, fenoprofen, fentanyl, fentiazac, fepradinol, feprazone, floctafenine, fluazacort, flucloronide, flufenamic acid, flumethasone, flunisolide, flunixin, flunoxaprofen, fluocinolone acetonide, fluocinonide, fluocinolone acetonide, fluocortin butyl, fluocortolone, fluoresone, fluorometholone, fluperolone, flupirtine, fluprednidene, fluprednisolone, fluproquazone, flurandrenolide, flurbiprofen, fluticasone, formocortal, fosfosal, gentisic acid, glafenine, glucametacin, glycol salicylate, guaiazulene, halcinonide, halobetasol, halometasone, haloprednone, heroin, hydrocodone, hydrocortamate, hydrocortisone, hydrocortisone acetate, hydrocortisone succinate, hydrocortisone hemisuccinate, hydrocortisone 21-lysinate, hydrocortisone cypionate, hydromorphone, hydroxypethidine, ibufenac, ibuprofen, ibuproxam, imidazole salicylate, indomethacin, indoprofen, isofezolac, isoflupredone, isoflupredone acetate, isoladol, isomethadone, isonixin, isoxepac, isoxicam, ketobemidone, ketoprofen, ketorolac, *p*-lactophenetide, lefetamine, levallorphan, levorphanol, levophenacyl-morphan, lofentanil, lonazolac, lornoxicam, loxoprofen, lysine acetylsalicylate, mazipredone, meclofenamic acid, medrysone, mefenamic acid, meloxicam, meperidine, meprednisone, meptazinol, mesalamine, metazocine, methadone, methotrimeprazine, methylprednisolone, methylprednisolone acetate, methylprednisolone sodium succinate, methylprednisolone suleptnate, metiazinic acid, metofoline, metopon, mofebutazone, mofezolac, mometasone, morazone, morphine, morphine hydrochloride, morphine sulfate, morpholine salicylate, myrophine, nabumetone, nalbuphine, nalorphine, 1-naphthyl salicylate, naproxen, narceine, nefopam, nicomorphine, nifenazone, niflumic acid, nimesulide, 5'-nitro-2'-propoxyacetanilide, norlevorphanol, normethadone, normorphine, norpipanone, olsalazine, opium, oxaceprol, oxametacine, oxaprozin, oxycodone, oxymorphone, oxyphenbutazone, papaveretum, paramethasone, paranyline, parsalmide, pentazocine, perisoxal, phenacetin, phenadoxone, phenazocine, phenazopyridine hydrochloride, phenocoll, phenoperidine, phenopyrazone, phenomorphan, phenyl acetylsalicylate, phenylbutazone, phenyl salicylate, phenyramidol, piketoprofen, piminodine, pipebuzone, piperylone, piprofen, pirazolac, piritramide, piroxicam, pranoprofen, prednicarbate, prednisolone, prednisone, prednival, prednylidene, proglumetacin, proheptazine, promedol, propacetamol, properidine, propiram, propoxyphene, propyphenazone, proquazone, protizinic acid, proxazole, ramifenazone, remifentanil, rimazolium metilsulfate, salacetamide, salicin, salicylamide, salicylamide o-acetic acid, salicylic acid, salicylsulfuric acid, safsalate, salverine, simetride, sufentanil, sulfasalazine, sulindac, superoxide dismutase, suprofen, suxibuzone, talniflumate, tenidap, tenoxicam, terofenamate, tetrandrine, thiazolinobutazone, tiaprofenic acid, tiaramide, tilidine, tinoridine, tixocortol, tolfenamic acid, tolmetin, tramadol, triamcinolone, triamcinolone acetonide, tropesin, viminol, xenbucin, ximoprofen, zaltoprofen and zomepirac.

8. The use of Claim 1 wherein the anti-inflammatory agent is a selective COX-2 inhibitor.

9. The use of Claim 8 wherein the selective COX-2 inhibitor is selected from the group consisting of deracoxib, parecoxib, celecoxib, valdecoxib, rofecoxib, etoricoxib, lumiracoxib, 2-(3,5-difluorophenyl)-3-[4-(methylsulfonyl)phenyl]-2-cyclopenten-1-one, (S)-6,8-dichloro-2-(trifluoromethyl)-2H-1-benzopyran-3-carboxylic acid, 2-(3,4-difluorophenyl)-4-(3-hydroxy-3-methyl-1-butoxy)-5-[4-(methylsulfonyl)phenyl]-3-(2H)-pyridazinone, 4-[5-(4-fluorophenyl)-3-(trifluoromethyl)-1H-pyrazol-1-yl]benzenesulfonamide, 4-[5-(phenyl)-3-(trifluoromethyl)-1H-pyrazol-1-yl]benzenesulfonamide, and salts thereof.

10. A pharmaceutical composition comprising a vehicle that comprises (a) an amphipathic oil that is water dispersible and ethanol insoluble, (b) microcrystalline wax and (c) a pharmaceutically acceptable non-aqueous carrier; said vehicle having stably dispersed therein an anti-inflammatory agent in an anti-inflammatorily effective amount.

11. The composition of Claim 10 wherein the anti-inflammatory agent is selected from the group consisting of aceclofenac, acemetacin, *e*-acetamidocaproic acid, acetaminophen, acetaminosalol, acetanilide, acetylsalicylic acid, *S*-adenosylmethionine, alclofenac, alclometasone, alfentanil, algestone, allylprodine, alminoprofen, aloxiprin, alphaprodine, aluminum bis(acetylsalicylate), amcinonide, amfenac, aminochlorthenoxazin, 3-amino-4-hydroxybutyric acid, 2-amino-4-picoline, aminopropylon, aminopyrine, amixetrine, ammonium salicylate, ampiroxicam, amtolmetin guacil, anileridine, antipyrine, antrafenine, apazone, beclomethasone, bendazac, benorylate, benoxaprofen, benzpiperylon, benzydamine, benzylmorphine, bermoprofen, betamethasone, betamethasone-17-valerate, bezitramide, α-bisabolol, bromfenac, p-bromoacetanilide, 5-bromosalicylic acid acetate, bromosaligenin, bucetin, bucloxic acid, bucolome, budesonide, bufexamac, bumadizon, buprenorphine, butacetin, butibufen, butorphanol, carbamazepine, carbiphene, carprofen, carsalam, chlorobutanol, chloroprednisone, chlorthenoxazin, choline salicylate, cinchophen, cinmetacin, ciramadol, clidanac, clobetasol, clocortolone, clometacin, clonitazene, clonixin, clopirac, cloprednol, clove, codeine, codeine methyl bromide, codeine phosphate, codeine sulfate, cortisone, cortivazol, cropropamide, crotethamide, cyclazocine, deflazacort, dehydrotestosterone, desomorphine, desonide, desoximetasone, dexamethasone, dexamethasone-21-isonicotinate, dexoxadrol, dextromoramide, dextropropoxyphene, deoxycorticosterone, dezocine, diampromide, diamorphone, diclofenac, difenamizole, difenpiramide, diflorasone, diflucortolone, diflunisal, difluprednate, dihydrocodeine, dihydrocodeinone enol acetate, dihydromorphine, dihydroxyaluminum acetylsalicylate, dimenoxadol, dimepheptanol, dimethylthiambutene, dioxaphetyl butyrate, dipipanone, diprocetyl, dipyrone, ditazol, droxicam, emorfazone, enfenamic acid, enoxolone, epirizole, eptazocine, etersalate, ethenzamide, ethoheptazine, ethoxazene, ethylmethylthiambutene, ethylmorphine, etodolac, etofenamate, etonitazene, eugenol, felbinac, fenbufen, fenclozic acid, fendosal, fenoprofen, fentanyl, fentiazac, fepradinol, feprazone, floctafenine, fluazacort, flucloronide, flufenamic acid, flumethasone, flunisolide, flunixin, flunoxaprofen, fluocinolone acetonide, fluocinonide, fluocinolone acetonide, fluocortin butyl, fluocortolone, fluoresone, fluorometholone, fluperolone, flupirtine, fluprednidene, fluprednisolone, fluproquazone, flurandrenolide, flurbiprofen, fluticasone, formocortal, fosfosal, gentisic acid, glafenine, glucametacin, glycol salicylate, guaiazulene, halcinonide, halobetasol, halometasone, haloprednone, heroin, hydrocodone, hydrocortamate, hydrocortisone, hydrocortisone acetate, hydrocortisone succinate, hydrocortisone hemisuccinate, hydrocortisone 21-lysinate, hydrocortisone cypionate, hydromorphone, hydroxypethidine, ibufenac, ibuprofen, ibuproxam, imidazole salicylate, indomethacin, indoprofen, isofezolac, isoflupredone, isoflupredone acetate, isoladol, isomethadone, isonixin, isoxepac, isoxicam, ketobemidone, ketoprofen, ketorolac, *p*-lactophenetide, lefetamine, levallorphan, levorphanol, levophenacyl-morphan, lofentanil, lonazolac, lornoxicam, loxoprofen, lysine acetylsalicylate, mazipredone, meclofenamic acid, medrysone, mefenamic acid, meloxicam, meperidine, meprednisone, meptazinol, mesalamine, metazocine, methadone, methotrimeprazine, methylprednisolone, methylprednisolone acetate, methylprednisolone sodium succinate, methylprednisolone suleptnate, metiazinic acid, metofoline, metopon, mofebutazone, mofezolac, mometasone, morazone, morphine, morphine hydrochloride, morphine sulfate, morpholine salicylate, myrophine, nabumetone, nalbuphine, nalorphine, 1-naphthyl salicylate, naproxen, narceine, nefopam, nicomorphine, nifenazone, niflumic acid, nimesulide, 5'-nitro-2'-propoxyacetanilide, norlevorphanol, normethadone, normorphine, norpipanone, olsalazine, opium, oxaceprol, oxametacine, oxaprozin, oxycodone, oxymorphone, oxyphenbutazone, papaveretum, paramethasone, paranyline, parsalmide, pentazocine, perisoxal, phenacetin, phenadoxone, phenazocine, phenazopyridine hydrochloride, phenocoll, phenoperidine, phenopyrazone, phenomorphan, phenyl acetylsalicylate, phenylbutazone, phenyl salicylate, phenyramidol, piketoprofen, piminodine, pipebuzone, piperylone, pirazolac, piritramide, piroxicam, pirprofen, pranoprofen, prednicarbate, prednisolone, prednisone, prednival, prednylidene, proglumetacin, proheptazine, promedol, propacetamol, properidine, propiram, propoxyphene, propyphenazone, proquazone, protizinic acid, proxazole, ramifenazone, remifentanil, rimazolium metilsulfate, salacetamide, salicin, salicylamide, salicylamide o-acetic acid, salicylic acid, salicylsulfuric acid, salsalate, salverine, simetride, sufentanil, sulfasalazine, sulindac, superoxide dismutase, suprofen, suxibuzone, talniflumate, tenidap, tenoxicam, terofenamate, tetrandrine, thiazolinobutazone, tiaprofenic acid, tiaramide, tilidine, tinoridine, tixocortol, tolfenamic acid, tolmetin, tramadol, triamcinolone, triamcinolone acetonide, tropesin, viminol, xenbucin, ximoprofen, zaltoprofen and zomepirac.

12. The composition of Claim 10 wherein the anti-inflammatory agent is a steroid.

13. The composition of Claim 10 wherein the anti-inflammatory agent is an NSAID.

14. The composition of Claim 10 wherein the anti-inflammatory agent is a selective COX-2 inhibitor.

15. The composition of Claim 14 wherein the selective COX-2 inhibitor is selected from the group consisting of deracoxib, parecoxib, celecoxib, valdecoxib, rofecoxib, etoricoxib, lumiracoxib, 2-(3,5-difluorophenyl)-3-[4-(methylsulfonyl)phenyl]-2-cyclopenten-1-one, (S)-6,8-dichloro-2-(trifluoromethyl)-2H-1-benzopyran-3-carboxylic acid, 2-(3,4-difluorophenyl)-4-(3-hydroxy-3-methyl-1-butoxy)-5-[4-(methylsulfonyl)phenyl]-3-(2H)-pyridazinone, 4-[5-(4-fluorophenyl)-3-(trifluoromethyl)-1H-pyrazol-1-yl]benzenesulfonamide, 4-[5-(phenyl)-3-(trifluoromethyl)-1H-pyrazol-1-yl]benzenesulfonamide, and salts thereof.

## Patentansprüche

1. Verwendung einer pharmazeutischen Zusammensetzung, die ein entzündungshemmendes Mittel zur Herstellung eines Medikaments zur Behandlung und/oder Prävention eines Entzündungszustands in einem Flüssigkeit enthaltenden Organ, das eine natürliche Außenöffnung aufweist, durch Verabreichen der Zusammensetzung an das Organ über die Außenöffnung, wobei die Zusammensetzung ferner ein Vehikel umfasst, das (a) ein amphipathisches Öl, das wasserdispergierbar und ethanolunlöslich ist, (b) ein mikrokristallines Wachs und (c) einen pharmazeutisch akzeptablen nichtwässrigen Träger umfasst.

2. Verwendung nach Anspruch 1, wobei das Flüssigkeit enthaltende Organ das Euter eines Milch produzierenden tierischen Lebewesens ist und wobei die Zusammensetzung durch intramammäre Infusion verabreicht wird.

3. Verwendung nach Anspruch 2, wobei der Entzündungszustand mit Mastitis in Verbindung steht.

4. Verwendung nach Anspruch 1, wobei das Flüssigkeit enthaltende Organ das Ohr eines Subjekts ist und wobei die Zusammensetzung durch Infusion in das Ohr verabreicht wird.

5. Verwendung nach Anspruch 4, wobei der Entzündungszustand mit einer Ohrenstörung in Verbindung steht, die ausgewählt ist aus der Gruppe von Otitis externa, Otitis media, Otorrhoe, akuter Mastoiditis, Infektionen in Verbindung mit chirurgischen Eingriffen am Ohr, Otosklerose, Otalgie, Ohrenschmerzen, Ohrenentzündung, Ohrenblutungen, Lermoyez-Anfall, Meniere-Krankheit, Neuronitis vestibularis, Lagerungsschwindel, Herpes Zoster oticus, Ramsey Hunt-Syndrom, viraler Neuronitis, Ganglionitis, Herpes geniculator, Labyrinthitis, die eitrige Labyrinthitis und virale endolymphatische Labyrinthitis umfasst, perilymphe Fisteln, Presbyakusis, arzneimittelinduzierter Ototoxizität, Acusticusneurinomen, Aerotitis media, infektiöser Myringitis, Myringitis bullosa, Ohrneoplasma, Plattenepithelkarzinom, Basalzellkarzinom, anderen Ohrkrebserkrankungen, präkanzerösen Ohrenzuständen, nicht-chromaffinen Paragangliomen, Chemodektomen, Glomus-jugulare-Tumoren, Glomus-tympanicum-Tumoren, Perichondritis, ekzematoider Ohrdermatitis, Otitis externa maligna, subperichondrealem Hämatom, Ceruminomen, Cerumen obturans, Epidermiszysten, Osteomen, Keloiden, Tinnitus, Vertigo, Trommelfellinfektion, Mittelohrentzündung, Ohrfurunkeln, Petrositis, Schallleitungs- und Schallempfindungsschwerhörigkeit, Epiduralabszess, Lateralsinusthrombose, subduralem Empyem, otitischem Hydrocephalus, Dandy-Syndrom, Myringitis bullosa, Otitis externa diffusa, Fremdkörpern, Keratosis obturans, Otomykose, Trauma, akuter Barotitis media, akuter Obstruktion der Eustach-Röhre, postoperativer Otalgie und damit verbundenen Komplikationen.

6. Verwendung nach Anspruch 4, wobei der Entzündungszustand mit einer Ohrenstörung in Verbindung steht, die aus der Gruppe von Otitis externa, Otitis media, Otorrhoe und Infektionen mit einer Entzündungskomponente, die mit einem chirurgischen Eingriff am Ohr in Verbindung steht, ausgewählt ist.

7. Verwendung nach Anspruch 1, wobei das entzündungshemmende Mittel ausgewählt ist aus der Gruppe von Aceclofenac, Acemetacin, e-Acetamidocapronsäure, Acetaminophen, Acetaminosalol, Acetanilid, Acetylsalicylsäure, S-Adenosylmethionin, Alclofenac, Alclometason, Alfentanil, Algeston, Allylprodin, Alminoprofen, Aloxiprin, Alphaprodin, Aluminiumbis(acetylsalicylat), Amcinonid, Amfenac, Aminochlorthenoxazin, 3-Amino-4-hydroxybuttersäure, 2-Amino-4-picolin, Aminopropylon, Aminopyrin, Amixetrin, Ammoniumsalicylat, Ampiroxicam, Amtolmetinguacil, Anileridin, Antipyrin, Antrafenin, Apazon, Beclomethason, Bendazac, Benorylat, Benoxaprofen, Benzpiperylon, Benzydamin, Benzylmorphin, Bermoprofen, Betamethason, Betamethason-17-valerat, Bezitramid, α-Bisabolol, Bromfenac, p-Bromacetanilid, 5-Bromsalicylsäureacetat, Bromsaligenin, Bucetin, Bucloxinsäure, Bucolom, Budesonid, Bufexamac, Bumadizon, Buprenorphin, Butacetin, Butibufen, Butorphanol, Carbamazepin, Carbiphen, Carprofen, Carsalam, Chlorbutanol, Chlorprednison, Chlorthenoxazin, Cholinsalicylat, Cinchophen, Cinmetacin, Ciramadol, Clidanac, Clobetasol, Clocortolon, Clometacin, Clonitazen, Clonixin, Clopirac, Cloprednol, Nelke, Codein, Codeinmethylbromid, Codeinphosphat, Codeinsulfat, Cortison, Cortivazol, Cropropamid, Crotethamid, Cyclazocin, Deflazacort, Dehydrotestosteron, Desomorphin, Desonid, Desoximetason, Dexamethason, Dexamethason-21-isonicotinat, Dexoxadrol, Dextromoramid, Dextropropoxyphen, Deoxycorticosteron, Dezocin, Diampromid, Diamorphon, Diclofenac, Difenamizol, Difenpiramid, Diflorason, Diflucortolon, Diflunisal, Difluprednat, Dihydrocodein, Dihydrocodeinonenolacetat, Dihydromorphin, Dihydroxyaluminiumacetylsalicylat, Dimenoxadol, Dimepheptanol, Dimethylthiambuten, Dioxaphetylbutyrat, Dipipanon, Diprocetyl, Dipyron, Ditazol, Droxicam, Emorfazon, Enfenaminsäure, Enoxolon, Epirizol, Eptazocin, Etersalat, Ethenzamid, Ethoheptazin, Ethoxazen, Ethylmethylthiambuten, Ethylmorphin, Etodolac, Etofenamat, Etonitazen, Eugenol, Felbinac, Fenbufen, Fenclozinsäure, Fendosal, Fenoprofen, Fentanyl, Fentiazac, Fepradinol, Feprazon, Floctafenin, Fluazacort, Flucloronid, Flufenaminsäure, Flumethason, Flunisolid, Flunixin, Flunoxaprofen, Fluocinolonacetonid, Fluocinonid, Fluocinolonacetonid, Fluocortinbutyl, Fluocortolon, Fluoreson, Fluorometholon, Fluperolon, Flupirtin, Flupredniden, Fluprednisolon, Fluproquazon, Flurandrenolid, Flurbiprofen, Fluticason, Formocortal, Fosfosal, Gentisinsäure, Glafenin, Glucametacin, Glycolsalicylat, Guaiazulen, Halcinonid, Halobetasol, Halometason, Haloprednon, Heroin, Hydrocodon, Hydrocortamat, Hydrocortison, Hydrocortisonacetat, Hydrocortisonsuccinat, Hydrocortisonhemisuccinat, Hydrocortison-21-lysinat, Hydrocortisoncypionat, Hydromorphon, Hydroxypethidin, Ibufenac, Ibuprofen, Ibuproxam, Imidazolsalicylat, Indomethacin, Indoprofen, Isofezolac, Isoflupredon, Isoflupredonacetat, Isoladol, Isomethadon, Isonixin, Isoxepac, Isoxicam, Ketobemidon, Ketoprofen, Ketorolac, p-Lactophenetid, Lefetamin, Levallorphan, Levorphanol, Levophenacyl-morphan, Lofentanil, Lonazolac, Lornoxicam, Loxoprofen, Lysinacetylsalicylat, Mazipredon, Meclofenaminsäure, Medryson, Mefenaminsäure, Meloxicam, Meperidin, Meprednison, Meptazinol, Mesalamin, Metazocin, Methadon, Methotrimeprazin, Methylprednisolon, Methylprednisolonacetat, Methylprednisolonnatriumsuccinat, Methylprednisolonsuleptnat, Metiazinsäure, Metofolin, Metopon, Mofebutazon, Mofezolac, Mometason, Morazon, Morphin, Morphinhydrochlorid, Morphinsulfat, Morpholinsalicylat, Myrophin, Nabumeton, Nalbuphin, Nalorphin, 1-Naphthylsalicylat, Naproxen, Narcein, Nefopam, Nicomorphin, Nifenazon, Nifluminsäure, Nimesulid, 5'-Nitro-2'-propoxyacetanilid, Norlevorphanol, Normethadon, Normorphin, Norpipanon, Olsalazin, Opium, Oxaceprol, Oxametacin, Oxaprozin, Oxycodon, Oxymorphon, Oxyphenbutazon, Papaveretum, Paramethason, Paranylin, Parsalmid, Pentazocin, Perisoxal, Phenacetin, Phenadoxon, Phenazocin, Phenazopyridinhydrochlorid, Phenocoll, Phenoperidin, Phenopyrazon, Phenomorphan, Phenylacetylsalicylat, Phenylbutazon, Phenylsalicylat, Phenyramidol, Piketoprofen, Piminodin, Pipebuzon, Piperylon, Piprofen, Pirazolac, Piritramid, Piroxicam, Pranoprofen, Prednicarbat, Prednisolon, Prednison, Prednival, Prednyliden, Proglumetacin, Proheptazin, Promedol, Propacetamol, Properidin, Propiram, Propoxyphen, Propyphenazon, Proquazon, Protizininsäure, Proxazol, Ramifenazon, Remifentanil, Rimazoliummetilsulfat, Salacetamid, Salicin, Salicylamid, Salicylamid-o-essigsäure, Salicylsäure, Salicylschwefelsäure, Salsalat, Salverin, Simetrid, Sufentanil, Sulfasalazin, Sulindac, Superoxiddismutase, Suprofen, Suxibuzon, Talniflumat, Tenidap, Tenoxicam, Terofenamat, Tetrandrin, Thiazolinobutazon, Tiaprofensäure, Tiaramid, Tilidin, Tinoridin, Tixocortol, Tolfenaminsäure, Tolmetin, Tramadol, Triamcinolon, Triamcinolonacetonid, Tropesin, Viminol, Xenbucin, Ximoprofen, Zaltoprofen und Zomepirac.

8. Verwendung nach Anspruch 1, wobei das entzündungshemmende Mittel ein selektiver COX-2-Inhibitor ist.

9. Verwendung nach Anspruch 8, wobei der selektive COX-2-Inhibitor aus der Gruppe von Deracoxib, Parecoxib, Celecoxib, Valdecoxib, Rofecoxib, Etoricoxib, Lumiracoxib, 2-(3,5-Difluorphenyl)-3-[4-(methylsulfonyl)phenyl]-2-cyclopenten-1-on, (S)-6,8-Dichlor-2-(trifluormethyl)-2H-1-benzopyran-3-carbonsäure, 2-(3,4-Difluorphenyl)-4-(3-hydroxy-3-methyl-1-butoxy)-5-[4-(methylsulfonyl)phenyl]-3-(2H)-pyridazinon, 4-[5-(4-Fluorphenyl)-3-(trifluormethyl)-1H-pyrazol-1-yl]benzolsulfonamid, 4-[5-(Phenyl)-3-(trifluormethyl)-1H-pyrazol-1-yl]benzolsulfonamid und Salzen derselben ausgewählt ist.

10. Pharmazeutische Zusammensetzung, die ein Vehikel umfasst, das (a) ein amphipathisches Öl, das wasserdispergierbar und ethanolunlöslich ist, (b) ein mikrokristallines Wachs und (c) einen pharmazeutisch akzeptablen nichtwässrigen Träger umfasst, wobei das Vehikel in diesem ein entzündungshemmendes Mittel in einer zur Entzündungshemmung wirksamen Menge stabil dispergiert aufweist.

11. Zusammensetzung nach Anspruch 10, wobei das entzündungshemmende Mittel ausgewählt ist aus der Gruppe von Aceclofenac, Acemetacin, e-Acetamidocapronsäure, Acetaminophen, Acetaminosalol, Acetanilid, Acetylsalicylsäure, S-Adenosylmethionin, Alclofenac, Alclometason, Alfentanil, Algeston, Allylprodin, Alminoprofen, Aloxiprin, Alphaprodin, Aluminiumbis(acetylsalicylat), Amcinonid, Amfenac, Aminochlorthenoxazin, 3-Amino-4-hydroxybuttersäure, 2-Amino-4-picolin, Aminopropylon, Aminopyrin, Amixetrin, Ammoniumsalicylat, Ampiroxicam, Amtolmetinguacil, Anileridin, Antipyrin, Antrafenin, Apazon, Beclomethason, Bendazac, Benorylat, Benoxaprofen, Benzpiperylon, Benzydamin, Benzylmorphin, Bermoprofen, Betamethason, Betamethason-17-valerat, Bezitramid, α-Bisabolol, Bromfenac, p-Bromacetanilid, 5-Bromsalicylsäureacetat, Bromsaligenin, Bucetin, Bucloxinsäure, Bucolom, Budesonid, Bufexamac, Bumadizon, Buprenorphin, Butacetin, Butibufen, Butorphanol, Carbamazepin, Carbiphen; Carprofen, Carsalam, Chlorbutanol, Chlorprednison, Chlorthenoxazin, Cholinsalicylat, Cinchophen, Cinmetacin, Ciramadol, Clidanac, Clobetasol, Clocortolon, Clometacin, Clonitazen, Clonixin, Clopirac, Cloprednol, Nelke, Codein, Codeinmethylbromid, Codeinphosphat, Codeinsulfat, Cortison, Cortivazol, Cropropamid, Crotethamid, Cyclazocin, Deflazacort, Dehydrotestosteron, Desomorphin, Desonid, Desoximetason, Dexamethason, Dexamethason-21-isonicotinat, Dexoxadrol, Dextromoramid, Dextropropoxyphen, Deoxycorticosteron, Dezocin, Diampromid, Diamorphon, Diclofenac, Difenamizol, Difenpiramid, Diflorason, Diflucortolon, Diflunisal, Difluprednat, Dihydrocodein, Dihydrocodeinonenolacetat, Dihydromorphin, Dihydroxyaluminiumacetylsalicylat, Dimenoxadol, Dimepheptanol, Dimethylthiambuten, Dioxaphetylbutyrat, Dipipanon, Diprocetyl, Dipyron, Ditazol, Droxicam, Emorfazon, Enfenaminsäure, Enoxolon, Epirizol, Eptazocin, Etersalat, Ethenzamid, Ethoheptazin, Ethoxazen, Ethylmethylthiambuten, Ethylmorphin, Etodolac, Etofenamat, Etonitazen, Eugenol, Felbinac, Fenbufen, Fenclozinsäure, Fendosal, Fenoprofen, Fentanyl, Fentiazac, Fepradinol, Feprazon, Floctafenin, Fluazacort, Flucloronid, Flufenaminsäure, Flumethason, Flunisolid, Flunixin, Flunoxaprofen, Fluocinolonacetonid, Fluocinonid, Fluocinolonacetonid, Fluocortinbutyl, Fluocortolon, Fluoreson, Fluorometholon, Fluperolon, Flupirtin, Flupredniden, Fluprednisolon, Fluproquazon, Flurandrenolid, Flurbiprofen, Fluticason, Formocortal, Fosfosal, Gentisinsäure, Glafenin, Glucametacin, Glycolsalicylat, Guaiazulen, Halcinonid, Halobetasol, Halometason, Haloprednon, Heroin, Hydrocodon, Hydrocortamat, Hydrocortison, Hydrocortisonacetat, Hydrocortisonsuccinat, Hydrocortisonhemisuccinat, Hydrocortison-21-lysinat, Hydrocortisoncypionat, Hydromorphon, Hydroxypethidin, Ibufenac, Ibuprofen, Ibuproxam, Imidazolsalicylat, Indomethacin, Indoprofen, Isofezolac, Isoflupredon, Isoflupredonacetat, Isoladol, Isomethadon, Isonixin, Isoxepac, Isoxicam, Ketobemidon, Ketoprofen, Ketorolac, p-Lactophenetid, Lefetamin, Levallorphan, Levorphanol, Levophenacyl-morphan, Lofentanil, Lonazolac, Lornoxicam, Loxoprofen, Lysinacetylsalicylat, Mazipredon, Meclofenaminsäure, Medryson, Mefenaminsäure, Meloxicam, Meperidin, Meprednison, Meptazinol, Mesalamin, Metazocin, Methadon, Methotrimeprazin, Methylprednisolon, Methylprednisolonacetat, Methylprednisolonnatriumsuccinat, Methylprednisolonsuleptnat, Metiazinsäure, Metofolin, Metopon, Mofebutazon, Mofezolac, Mometason, Morazon, Morphin, Morphinhydrochlorid, Morphinsulfat, Morpholinsalicylat, Myrophin, Nabumeton, Nalbuphin, Nalorphin, 1-Naphthylsalicylat, Naproxen, Narcein, Nefopam, Nicomorphin, Nifenazon, Nifluminsäure, Nimesulid, 5'-Nitro-2'-propoxyacetanilid, Norlevorphanol, Normethadon, Normorphin, Norpipanon, Olsalazin, Opium, Oxaceprol, Oxametacin, Oxaprozin, Oxycodon, Oxymorphon, Oxyphenbutazon, Papaveretum, Paramethason, Paranylin, Parsalmid, Pentazocin, Perisoxal, Phenacetin, Phenadoxon, Phenazocin, Phenazopyridinhydrochlorid, Phenocoll, Phenoperidin, Phenopyrazon, Phenomorphan, Phenylacetylsalicylat, Phenylbutazon, Phenylsalicylat, Phenyramidol, Piketoprofen, Piminodin, Pipebuzon, Piperylon, Pirazolac, Piritramid, Piroxicam, Pirprofen, Pranoprofen, Prednicarbat, Prednisolon, Prednison, Prednival, Prednyliden, Proglumetacin, Proheptazin, Promedol, Propacetamol, Properidin, Propiram, Propoxyphen, Propyphenazon, Proquazon, Protizininsäure, Proxazol, Ramifenazon, Remifentanil, Rimazoliummetilsulfat, Salacetamid, Salicin, Salicylamid, Salicylamid-o-essigsäure, Salicylsäure, Salicylschwefelsäure, Salsalat, Salverin, Simetrid, Sufentanil, Sulfasalazin, Sulindac, Superoxiddismutase, Suprofen, Suxibuzon, Talniflumat, Tenidap, Tenoxicam, Terofenamat, Tetrandrin, Thiazolinobutazon, Tiaprofensäure, Tiaramid, Tilidin, Tinoridin, Tixocortol, Tolfenaminsäure, Tolmetin, Tramadol, Triamcinolon, Triamcinolonacetonid, Tropesin, Viminol, Xenbucin, Ximoprofen, Zaltoprofen und Zomepirac.

12. Zusammensetzung nach Anspruch 10, wobei das entzündungshemmende Mittel ein Steroid ist.

13. Zusammensetzung nach Anspruch 10, wobei das entzündungshemmende Mittel ein NSAID ist.

14. Zusammensetzung nach Anspruch 10, wobei das entzündungshemmende Mittel ein selektiver COX-2-Inhibitor ist.

15. Zusammensetzung nach Anspruch 14, wobei der selektive COX-2-Inhibitor aus der Gruppe von Deracoxib, Parecoxib, Celecoxib, Valdecoxib, Rofecoxib, Etoricoxib, Lumiracoxib, 2-(3,5-Difluorphenyl)-3-[4-(methylsulfonyl)phenyl]-2-cyclopenten-1-on, (S)-6,8-Dichlor-2-(trifluormethyl)-2H-1-benzopyran-3-carbonsäure, 2-(3,4-Difluorphenyl)-4-(3-hydroxy-3-methyl-1-butoxy)-5-[4-(methylsulfonyl)phenyl]-3-(2H)-pyridazinon, 4-[5-(4-Fluorphenyl)-3-(trifluormethyl)-1H-pyrazol-1-yl]benzolsulfonamid, 4-[5-(Phenyl)-3-(trifluormethyl)-1H-pyrazol-1-yl]benzolsulfonamid und Salzen derselben ausgewählt ist.

## Revendications

1. Utilisation d'une composition pharmaceutique comprenant un agent anti-inflammatoire pour la production d'un médicament destiné au traitement et/ou à la prévention d'un état inflammatoire dans un organe contenant un fluide, ayant un orifice extérieur naturel, en administrant la composition à l'organe par l'orifice extérieur, ladite composition comprenant en outre un véhicule qui comprend (a) une huile amphipathique qui est dispersible dans l'eau et insoluble dans l'éthanol, (b) une cire microcristalline et (c) un support non aqueux pharmaceutiquement acceptable.

2. Utilisation suivant la revendication 1, dans laquelle l'organe contenant un fluide est un pis d'un animal producteur de lait, et dans laquelle la composition est administrée par instillation intramammaire.

3. Utilisation suivant la revendication 2, dans laquelle l'état inflammatoire est associé à la mastite.

4. Utilisation suivant la revendication 1, dans laquelle l'organe contenant un fluide est une oreille d'un sujet, et dans laquelle la composition est administrée par instillation dans l'oreille.

5. Utilisation suivant la revendication 4, dans laquelle l'état inflammatoire est associé à un trouble auditif choisi dans le groupe consistant en l'otite externe, l'otite moyenne, l'otorrhée, la mastoïdite aiguë, des infections liées à des opérations chirurgicales auriculaires, l'otosclérose, l'otalgie, la douleur auriculaire, l'inflammation auriculaire, le saignement auriculaire, le syndrome de Lermoyez, la maladie de Ménière, la neuronite vestibulaire, le vertige phonolithique paroxystique bénin, l'Herpes zoster auriculaire, le syndrome de Ramsay Hunt, la neuronite virale, la ganglionite, l'herpès géniculé, la labyrinthite, y compris la labyrinthite purulente et la labyrinthite endolymphatique virale, les fistules périlymphatiques, la presbyacousie, l'autotoxicité induite par un médicament, les neuromes acoustiques, l'otite barotraumatique moyenne, la myringite infectieuse, la myringite bulleuse, un néoplasme auriculaire, un carcinome à cellules squameuses, un carcinome à cellules basales, d'autres cancers auriculaires, des affections auriculaires précancéreuses, des paragangliomes non chromaffines, des chémodectomes, des tumeurs du glomus jugulaire, des tumeurs du glomus tympanique, la périchondrite, la dermatite eczématoïde auriculaire, l'otite externe maligne, un hématome sous-périchondral, des céruminomes, des bouchons de cérumen, des kystes sébacés, des ostéomes, des chéloïdes, les bourdonnements de l'oreille, les vertiges, une infection de la membrane tympanique, la tympanite, les furoncles auriculaires, la pétrosite, une perte d'audition conductrice et de perception, un abcès épidural, une thrombose du sinus latéral, un empyème sous-dural, l'hydrocéphalie auriculaire, le syndrome de Dandy, la tympanite bulleuse, l'otite externe diffuse, la présence de corps étrangers, la présence d'un bouchon épidermique, l'otomycose, un traumatisme, l'otite barotraumatique aiguë, une obstruction aiguë des trompes d'Eustache, l'otalgie postchirurgicale et les complications qui y sont associées.

6. Utilisation suivant la revendication 4, dans laquelle l'état inflammatoire est associé à un trouble auriculaire choisi dans le groupe consistant en l'otite externe, l'otite moyenne, l'otorrhée et des infections ayant une composante inflammatoire qui sont liées à une opération chirurgicale auriculaire.

7. Utilisation suivant la revendication 1, dans laquelle l'agent anti-inflammatoire est choisi dans le groupe consistant en l'acéclofénac, l'acémétacine, l'acide ε-acétamidocaproïque, l'acétaminophène, l'acétaminosalol, l'acétanilide, l'acide acétylsalicylique, la S-adénosylméthionine, l'alclofénac, l'alclométasone, l'alfentanil, l'algestone, l'allylprodine, l'alminoprofène, l'aloxiprine, l'alphaprodine, le bis(acétylsalicylate) d'aluminium, l'amcinonide, l'amfénac, l'aminochlorthénoxazine, l'acide 3-amino-4-hydroxybutyrique, la 2-amino-4-picoline, l'aminopropylon, l'aminopyrine, l'amixétrine, le salicylate d'ammonium, l'ampiroxicam, l'amtolmétine-guacile, l'aniléridine, l'antipyrine, l'antrafénine, l'apazone, la béclométhasone, le bendazac, le bénorylate, le bénoxaprofène, le benzpipérylon, la benzydamine, la benzylmorphine, le bermoprofène, la bétaméthasone, le 17-valérate de bétaméthasone, le bézitramide, l'a-bisabolol, le bromfénac, le p-bromoacétanilide, l'acétate d'acide 5-bromosalicylique, la bromosaligénine, la bucétine, l'acide bucloxique, le bucolome, le budésonide, le bufexamac, le bumadizon, la buprénorphine, la butacétine, le butibufène, le butorphanol, la carbamazépine, le carbiphène, le carprofène, le carsalam, le chlorobutanol, la chloroprednisone, la chlorthénoxazine, le salicylate de choline, le cinchophène, la cinmétacine, le ciramadol, le clidanac, le clobétasol, la clocortolone, la clométacine, le clonitazène, la clonixine, le clopirac, le cloprednol, l'essence de girofle, la codéine, le bromure méthylique de codéine, le phosphate de codéine, le sulfate de codéine, la cortisone, le cortivazol, le cropropamide, le crotéthamide, la cyclazocine, le déflazacort, la déhydrotestostérone, la désomorphine, le désonide, la désoximétasone, la dexaméthasone, le 21-isonicotinate de dexaméthasone, le dexoxadrol, le dextromoramide, le dextropropoxyphène, la désoxycorticostérone, la dézocine, le diampromide, la diamorphone, le diclofénac, le difénamizole, le difenpiramide, la diflorasone, la diflucortolone, le diflunisal, le difluprednate, la dihydrocodéine, l'acétate d'énol de dihydrocodéinone, la dihydromorphine, l'acétylsalicylate de dihydroxyaluminium, le diménoxadol, le dimépheptanol, le diméthylthiambutène, le butyrate de dioxaphétyl, la dipipanone, le diprocétyle, la dipyrone, le ditazol, le droxicam, l'émorfazone, l'acide enfénamique, l'énoxolone, l'épirizole, l'eptazocine, l'étersalate, l'éthenzamide, l'éthoheptazine, l'éthoxazène, l'éthylméthylthiambutène, l'éthylmorphine, l'étodolac, l'étofénamate, l'étonitazène, l'eugénol, le felbinac, le fenbufène, l'acide fenclozique, le fendosal, le fénoprofène, le fentanyl, le fentiazac, le fépradinol, la féprazone, la floctafénine, le fluazacort, le flucloronide, l'acide flufénamique, la fluméthasone, le flunisolide, la flunixine, le flunoxaprofène, le fluocinolone acétonide, le fluocinonide, le fluocinolone acétonide, le fluocortine-butyl, la fluocortolone, la fluorésone, la fluorométholone, la flupérolone, la flupirtine, le fluprednidène, la fluprednisolone, la fluproquazone, le flurandrénolide, le flurbiprofène, la fluticasone, le formocortal, le fosfosal, l'acide gentisique, la glafénine, la glucamétacine, le salicylate de glycol, le guaïazulène, l'halcinonide, l'halobétasol, l'halométasone, l'haloprednone, l'héroïne, l'hydrocodone, l'hydrocortamate, l'hydrocortisone, l'acétate d'hydrocortisone, le succinate d'hydrocortisone, l'hémisuccinate d'hydrocortisone, le 21-lysinate d'hydrocortisone, le cypionate d'hydrocortisone, l'hydromorphone, l'hydroxypéthidine, l'ibufénac, l'ibuprofène, l'ibuproxam, le salicylate d'imidazole, l'indométhacine, l'indoprofène, l'isofézolac, l'isofluprédone, l'acétate d'isofluprédone, l'isoladol, l'isométhadone, l'isonixine, l'isoxépac, l'isoxicam, le kétobémidone, le kétoprofène, le kétorolac, le p-lactophénétide, la léfétamine, le lévallorphane, le lévorphanol, le lévophénacyl-morphane, le lofentanil, le lonazolac, le lornoxicam, le loxoprofène, l'acétylsalicylate de lysine, la maziprédone, l'acide méclofénamique, la médrysone, l'acide méfénamique, le méloxicam, la mépéridine, la méprednisone, le meptazinol, la mésalamine, la métazocine, la méthadone, la méthotriméprazine, la méthylprednisolone, l'acétate de méthylprednisolone, le succinate sodique de méthylprednisolone, le suleptnate de méthylprednisolone, l'acide métiazinique, la métofoline, le métopon, la mofébutazone, le mofézolac, la mométasone, la morazone, la morphine, le chlorhydrate de morphine, le sulfate de morphine, le salicylate de morphine, la myrophine, la nabumétone, la nalbuphine, la nalorphine, le salicylate de 1-naphtyle, le naproxène, la narcéine, le néfopam, la nicomorphine, la nifénazone, l'acide niflumique, le nimésulide, le 5'-nitro-2'-propoxyacétanilide, le norlévorphanol, la norméthadone, la normorphine, la norpinanone, l'olsalazine, l'opium, l'oxacéprol, l'oxamétacine, l'oxaprozine, l'oxycodone, l'oxymorphone, l'oxyphenbutazone, le papavérétum, la paraméthasone, la paranyline, le parsalmide, la pentazocine, le périsoxal, la phénacétine, la phénadoxone, la phénazocine, le chlorhydrate de phénazopyridine, le phénocoll, la phénopéridine, la phénopyrazone, le phénomorphane, l'acétylsalicylate de phényle, la phénylbutazone, le salicylate de phényle, le phényramidol, le pikétoprofène, la piminodine, la pipébuzone, la pipérylone, le piprofène, le pirazolac, le piritramide, le piroxicam, le pranoprofène, le prednicarbate, la prednisolone, la prednisone, le prednival, le prednylidène, la proglumétacine, la proheptazine, le promédol, le propacétamol, la propéridine, le propiram, le propoxyphène, la propyphénazone, la proquazone, l'acide protizinique, le proxazole, la ramifénazone, le rémifentanil, le méthylsulfate de rimazolium, le salacétamide, la salicine, le salicylamide, l'acide salicylamide-o-acétique, l'acide salicylique, l'acide salicylsulfurique, le salsalate, la salvérine, le simétride, le sufentanil, la sulfasalazine, le sulindac, la superoxyde-dismutase, le suprofène, la suxibuzone, la talniflumate, le ténidap, le ténoxicam, le térofénamate, la tétrandrine, la thiazolinobutazone, l'acide tiaprofénique, le tiaramide, la tilidine, la tinoridine, le tixocortol, l'acide tolfénamique, la tolmétine, le tramadol, la triamcinolone, le triamcinolone acétonide, la tropésine, le viminol, la xenbucine, le ximoprofène, le zaltoprofène et le zomépirac.

8. Utilisation suivant la revendication 1, dans laquelle l'agent anti-inflammatoire est un inhibiteur de COX-2 sélectif.

9. Utilisation suivant la revendication 8, dans laquelle l'inhibiteur de COX-2 sélectif est choisi dans le groupe consistant en déracoxib, parécoxib, célécoxib, valdécoxib, rofécoxib, étoricoxib, lumiracoxib, 2-(3,5-difluorophényl)-3-[4-(méthylsulfonyl)phényl]-2-cyclopentène-1-one, acide (S)-6,8-dichloro-2-(trifluorométhyl)-2H-1-benzopyranne-3-carboxylique, 2-(3,4-difluorophényl)-4-(3-hydroxy-3-méthyl-1-butoxy) -5-[4-(méthylsulfonyl)phényl]-3-(2H)-pyridazinone, 4-[5-(4-fluorophényl)-3-(trifluorométhyl)-1H-pyrazole-1-yl]benzènesulfonamide, 4-[5-(phényl)-3-(trifluorométhyl)-1H-pyrazole-1-yl]benzènesulfonamide et leurs sels.

10. Composition pharmaceutique comprenant un véhicule qui comprend (a) une huile amphipathique qui est dispersible dans l'eau et insoluble dans l'éthanol, (b) une cire microcristalline et (c) un support non aqueux pharmaceutiquement acceptable ; ledit véhicule renfermant à l'état dispersé de manière stable un agent anti-inflammatoire en une quantité efficace du point de vue anti-inflammatoire.

11. Composition suivant la revendication 10, dans laquelle l'agent anti-inflammatoire est choisi dans le groupe consistant en l'acéclofénac, l'acémétacine, l'acide s-acétamidocaproïque, l'acétaminophène, l'acétaminosalol, l'acétanilide, l'acide acétylsalicylique, la S-adénosylméthionine, l'alclofénac, l'alclométasone, l'alfentanil, l'algestone, l'allylprodine, l'alminoprofène, l'aloxiprine, l'alphaprodine, le bis(acétylsalicylate) d'aluminium, l'amcinonide, l'amfénac, l'aminochlorthénoxazine, l'acide 3-amino-4-hydroxybutyrique, la 2-amino-4-picoline, l'aminopropylon, l'aminopyrine, l'amixétrine, le salicylate d'ammonium, l'ampiroxicam, l'amtolmétine-guacile, l'aniléridine, l'antipyrine, l'antrafénine, l'apazone, la béclométhasone, le bendazac, le bénorylate, le bénoxaprofène, le benzpipérylon, la benzydamine, la benzylmorphine, le bermoprofène, la bétaméthasone, le 17-valérate de bétaméthasone, le bézitramide, l'α-bisabolol, le bromfénac, le p-bromoacétanilide, l'acétate d'acide 5-bromosalicylique, la bromosaligénine, la bucétine, l'acide bucloxique, le bucolome, le budésonide, le bufexamac, le bumadizon, la buprénorphine, la butacétine, le butibufène, le butorphanol, la carbamazépine, le carbiphène, le carprofène, le carsalam, le chlorobutanol, la chloroprednisone, la chlorthénoxazine, le salicylate de choline, le cinchophène, la cinmétacine, le ciramadol, le clidanac, le clobétasol, la clocortolone, la clométacine, le clonitazène, la clonixine, le clopirac, le cloprednol, l'essence de girofle, la codéine, le bromure méthylique de codéine, le phosphate de codéine, le sulfate de codéine, la cortisone, le cortivazol, le cropropamide, le crotéthamide, la cyclazocine, le déflazacort, la déhydrotestostérone, la désomorphine, le désonide, la désoximétasone, la dexaméthasone, le 21-isonicotinate de dexaméthasone, le dexoxadrol, le dextromoramide, le dextropropoxyphène, la désoxycorticostérone, la dézocine, le diampromide, la diamorphone, le diclofénac, le difénamizole, le difenpiramide, la diflorasone, la diflucortolone, le diflunisal, le difluprednate, la dihydrocodéine, l'acétate d'énol de dihydrocodéinone, la dihydromorphine, l'acétylsalicylate de dihydroxyaluminium, le diménoxadol, le dimépheptanol, le diméthylthiambutène, le butyrate de dioxaphétyl, la dipipanone, le diprocétyle, la dipyrone, le ditazol, le droxicam, l'émorfazone, l'acide enfénamique, l'énoxolone, l'épirizole, l'eptazocine, l'étersalate, l'éthenzamide, l'éthoheptazine, l'éthoxazène, l'éthylméthylthiambutène, l'éthylmorphine, l'étodolac, l'étofénamate, l'étonitazène, l'eugénol, le felbinac, le fenbufène, l'acide fenclozique, le fendosal, le fénoprofène, le fentanyl, le fentiazac, le fépradinol, la féprazone, la floctafénine, le fluazacort, le flucloronide, l'acide flufénamique, la fluméthasone, le flunisolide, la flunixine, le flunoxaprofène, le fluocinolone acétonide, le fluocinonide, le fluocinolone acétonide, le fluocortinebutyl, la fluocortolone, la fluorésone, la fluorométholone, la flupérolone, la flupirtine, le fluprednidène, la fluprednisolone, la fluproquazone, le flurandrénolide, le flurbiprofène, la fluticasone, le formocortal, le fosfosal, l'acide gentisique, la glafénine, la glucamétacine, le salicylate de glycol, le guaïazulène, l'halcinonide, l'halobétasol, l'halométasone, l'haloprednone, l'héroïne, l'hydrocodone, l'hydrocortamate, l'hydrocortisone, l'acétate d'hydrocortisone, le succinate d'hydrocortisone, l'hémisuccinate d'hydrocortisone, le 21-lysinate d'hydrocortisone, le cypionate d'hydrocortisone, l'hydromorphone, l'hydroxypéthidine, l'ibufénac, l'ibuprofène, l'ibuproxam, le salicylate d'imidazole, l'indométhacine, l'indoprofène, l'isofézolac, l'isofluprédone, l'acétate d'isofluprédone, l'isoladol, l'isométhadone, l'isonixine, l'isoxépac, l'isoxicam, le kétobémidone, le kétoprofène, le kétorolac, le p-lactophénétide, la léfétamine, le lévallorphane, le lévorphanol, le lévophénacyl-morphane, le lofentanil, le lonazolac, le lornoxicam, le loxoprofène, l'acétylsalicylate de lysine, la maziprédone, l'acide méclofénamique, la médrysone, l'acide méfénamique, le méloxicam, la mépéridine, la méprednisone, le meptazinol, la mésalamine, la métazocine, la méthadone, la méthotriméprazine, la méthylprednisolone, l'acétate de méthylprednisolone, le succinate sodique de méthylprednisolone, le suleptnate de méthylprednisolone, l'acide métiazinique, la métofoline, le métopon, la mofébutazone, le mofézolac, la mométasone, la morazone, la morphine, le chlorhydrate de morphine, le sulfate de morphine, le salicylate de morphine, la myrophine, la nabumétone, la nalbuphine, la nalorphine, le salicylate de 1-naphtyle, le naproxène, la narcéine, le néfopam, la nicomorphine, la nifénazone, l'acide niflumique, le nimésulide, le 5'-nitro-2'-propoxyacétanilide, le norlévorphanol, la norméthadone, la normorphine, la norpinanone, l'olsalazine, l'opium, l'oxacéprol, l'oxamétacine, l'oxaprozine, l'oxycodone, l'oxymorphone, l'oxyphenbutazone, le papavérétum, la paraméthasone, la paranyline, le parsalmide, la pentazocine, le périsoxal, la phénacétine, la phénadoxone, la phénazocine, le chlorhydrate de phénazopyridine, le phénocoll, la phénopéridine, la phénopyrazone, le phénomorphane, l'acétylsalicylate de phényle, la phénylbutazone, le salicylate de phényle, le phényramidol, le pikétoprofène, la piminodine, la pipébuzone, la pipérylone, le pirazolac, le piritramide, le piroxicam, le le pirprofène, le pranoprofène, le prednicarbate, la prednisolone, la prednisone, le prednival, le prednylidène, la proglumétacine, la proheptazine, le promédol, le propacétamol, la propéridine, le propiram, le propoxyphène, la propyphénazone, la proquazone, l'acide protizinique, le proxazole, la ramifénazone, le rémifentanil, le méthylsulfate de rimazolium, le salacétamide, la salicine, le salicylamide, l'acide salicylamide-o-acétique, l'acide salicylique, l'acide salicylsulfurique, le salsalate, la salvérine, le simétride, le sufentanil, la sulfasalazine, le sulindac, la superoxyde-dismutase, le suprofène, la suxibuzone, la talniflumate, le ténidap, le ténoxicam, le térofénamate, la tétrandrine, la thiazolinobutazone, l'acide tiaprofénique, le tiaramide, la tilidine, la tinoridine, le tixocortol, l'acide tolfénamique, la tolmétine, le tramadol, la triamcinolone, le triamcinolone acétonide, la tropésine, le viminol, la xenbucine, le ximoprofène, le zaltoprofène et le zomépirac.

12. Composition suivant la revendication 10, dans laquelle l'agent anti-inflammatoire est un stéroïde.

13. Composition suivant la revendication 10, dans laquelle l'agent anti-inflammatoire est un médicament anti-inflammatoire non stéroïdien.

14. Composition suivant la revendication 10, dans laquelle l'agent anti-inflammatoire est un inhibiteur de COX-2 sélectif.

15. Composition suivant la revendication 14, dans laquelle l'inhibiteur de COX-2 sélectif est choisi dans le groupe consistant en déracoxib, parécoxib, célécoxib, valdécoxib, rofécoxib, étoricoxib, lumiracoxib, 2-(3,5-difluorophenyl)-3-[4-(méthylsulfonyl)phényl]-2-cyclopentène-1-one, acide (S)-6,8-dichloro-2-(trifluorométhyl)-2H-1-benzopyranne-3-carboxylique, 2-(3,4-difluorophényl)-4-(3-hydroxy-3-méthyl-1-butoxy)-5-[4-(méthylsulfonyl)phényl]-3-(2H)-pyridazinone, 4-[5-(4-fluorophényl)-3-(trifluorométhyl)-1H-pyrazole-1-yl]benzènesulfonamide, 4-[5-(phényl)-3-(trifluorométhyl)-1H-pyrazole-1-yl]benzènesulfonamide et leurs sels.
